(19) Europäisches Patentamt
European Patent Office
Office européen des brevets

(11) **EP 3 592 727 B1**

(12) **EUROPÄISCHE PATENTSCHRIFT**

(45) Veröffentlichungstag und Bekanntmachung des Hinweises auf die Patenterteilung:
**03.04.2024 Patentblatt 2024/14**

(21) Anmeldenummer: **18709001.4**

(22) Anmeldetag: **05.03.2018**

(51) Internationale Patentklassifikation (IPC):
**C07C 201/08** (2006.01)     **C07C 201/16** (2006.01)
**C07C 205/06** (2006.01)

(52) Gemeinsame Patentklassifikation (CPC):
(C-Sets verfügbar)
**C07C 201/08; C07C 201/16**          (Forts.)

(86) Internationale Anmeldenummer:
**PCT/EP2018/055376**

(87) Internationale Veröffentlichungsnummer:
**WO 2018/162427 (13.09.2018 Gazette 2018/37)**

(54) **VERFAHREN ZUR HERSTELLUNG VON NITROBENZOL**

METHOD FOR PRODUCING NITROBENZENE

PROCÉDÉ DE FABRICATION DE NITROBENZÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.03.2017 EP 17159723**

(43) Veröffentlichungstag der Anmeldung:
**15.01.2020 Patentblatt 2020/03**

(73) Patentinhaber: **Covestro Deutschland AG
51373 Leverkusen (DE)**

(72) Erfinder:
• **CHAN, Denise
40219 Düsseldorf (DE)**
• **KNAUF, Thomas
41542 Dormagen (DE)**

• **MÜNNIG, Jürgen
41564 Kaarst (DE)**

(74) Vertreter: **Levpat
c/o Covestro AG
Gebäude 4825
51365 Leverkusen (DE)**

(56) Entgegenhaltungen:
**WO-A1-2014/016290     WO-A1-2014/177450
WO-A1-2015/197521     DE-A1- 2 821 571**

(52) Gemeinsame Patentklassifikation (CPC): (Forts.)

C-Sets
**C07C 201/08, C07C 205/06;**
**C07C 201/16, C07C 205/06**

C-Sets
**C07C 201/08, C07C 205/06;**
**C07C 201/16, C07C 205/06**

**Beschreibung**

Die Erfindung betrifft ein Verfahren zur kontinuierlichen Herstellung von Nitrobenzol durch Nitrierung von Benzol mit Salpetersäure und Schwefelsäure, bei welchem Lastwechsel (d. h. im Sinne der vorliegenden Erfindung eine Verringerung der dem Prozess pro Zeitspanne zugeführten Menge an Salpetersäure) besonders vorteilhaft ausgestaltet werden. Die vorliegende Erfindung stellt ein Verfahren bereit, bei welchem im Falle einer Lasterniedrigung das Verhältnis der pro Zeitspanne zugeführten Massen an Salpetersäure und Schwefelsäure im Vergleich zu diesem Verhältnis vor dem Lastwechsel signifikant erniedrigt wird.

Nitrobenzol (auch Mononitrobenzol, MNB, genannt) ist ein wichtiges Zwischenprodukt der chemischen Industrie, das insbesondere zur Herstellung von Anilin (ANL) und damit auch zur Herstellung der Di- und Polyisocyanate der Diphenylmethanreihe (MDI) und den darauf basierenden Polyurethanen benötigt wird.

Die heute gängigen Verfahren zur Nitrierung von Benzol mit Salpetersäure zu Nitrobenzol entsprechen im Wesentlichen dem Konzept der adiabatisch betriebenen Nitrierung von Benzol durch ein Gemisch von Schwefel- und Salpetersäure, welches im Allgemeinen als Mischsäure bezeichnet wird. Ein solches Verfahren ist beispielsweise in EP 0 436 443 B1, EP 0 771 783 B1 und US 6 562 247 B2 beschrieben. Den beschriebenen adiabatisch betriebenen Verfahren ist gemein, dass die Ausgangsstoffe Benzol und Salpetersäure in einem großen Überschuss an Schwefelsäure zur Reaktion gebracht werden, welche die frei werdende Reaktionswärme und das bei der Reaktion gebildete Wasser aufnimmt. Die Reaktionsführung erfolgt im Allgemeinen so, dass die Salpetersäure und Schwefelsäure zur sogenannten Nitriersäure (auch Mischsäure genannt) vereint werden. Benzol wird in diese Nitriersäure hinein dosiert. Die Reaktionsprodukte sind im Wesentlichen Wasser und Nitrobenzol. In der Nitrierreaktion wird Benzol, bezogen auf die molare Salpetersäuremenge, mindestens in stöchiometrischer Menge, aber bevorzugt in 2%igem bis 10%igem Überschuss eingesetzt. Das in den Reaktionsapparaten gebildete und in Phasentrennapparaten von der Säurephase abgetrennte Rohnitrobenzol wird gemäß dem Stand der Technik einer Wäsche und einer destillativen Aufarbeitung unterzogen, wie beispielsweise in EP 1 816 117 A1 (Seite 2, Zeile 26 bis 42), US 4,091,042 (siehe oben) oder US 5,763,697 beschrieben. Charakteristisch für diese Aufarbeitung ist, dass nicht umgesetztes überschüssiges Benzol nach der Wäsche von Nitrobenzol in einer abschließenden Destillation abgetrennt wird und als sog. "Rückbenzol" wieder in der Nitrierreaktion eingesetzt wird. Hierzu wird es mit frisch zugeführtem Benzol ("Frischbenzol") zum sog. "Einsatzbenzol" vermischt. Die im Wesentlichen aus Schwefelsäure bestehende Säurephase wird in einem sog. Blitzverdampfer aufkonzentriert und weitestgehend von Organika befreit. Die auf diese Weise aufkonzentrierte Schwefelsäure wird als sog. Kreislaufschwefelsäure in die Nitrierung zurückgefahren.

DE 28 21 571 A1 betrifft ein kontinuierliches adiabatisches Nitrierverfahren bei dem man einen Reaktionsstrom aus einer Mischsäure mit etwa 3 bis 7,5 % Salpetersäure, etwa 58,5 bis 66,5 % Schwefelsäure und etwa 28 bis 37 % Wasser und einen Reaktionsstrom mit bis zu einem etwa 10-prozentigen stöchiometrischen Überschuss an Benzol kontinuierlich miteinander vermischt und unter kräftigem Rühren bei einer Temperatur von etwa 80 bis 120 °C bei überatmosphärischem Druck derart umsetzt, dass die Reaktionstemperatur etwa 145 °C nicht übersteigt, wodurch unter praktisch vollständiger Umwandlung der Salpetersäure Mononitrobenzol mit einem Gehalt von weniger als etwa 500 ppm an Dinitrobenzol gebildet wird.

WO 2015/197521 A1 beschreibt ein Verfahren zur kontinuierlichen Herstellung von Nitrobenzol durch Nitrierung von Benzol mit einem Gemisch aus Salpetersäure und Schwefelsäure, bei dem während eines Produktionsstillstandes nicht die ganze Produktionsanlage abgefahren wird, sondern die Produktionsanlage ganz oder zumindest teilweise "im Kreis" gefahren wird. Des Weiteren betrifft die Anmeldung eine Anlage zur Herstellung von Nitrobenzol und ein Verfahren zum Betrieb einer Anlage zur Herstellung von Nitrobenzol.

WO 2014/177450 A1 beschreibt ein kontinuierlich betriebenes adiabates Verfahren zur Herstellung von Nitrobenzol durch Nitrierung von Benzol mit Salpetersäure und Schwefelsäure, bei dem die nach erfolgter Nitrierung und Abtrennung des rohen Nitrobenzols von der wässrigen Phase erhaltene verdünnte Schwefelsäure zum Zwecke der Wiederverwendung in der Nitrierung aufkonzentriert und nach ihrer Aufkonzentrierung mindestens eine Minute, bevor sie wieder mit frischer Salpetersäure in Kontakt tritt, so mit einem Oxidationsmittel versetzt wird, dass sich eine Konzentration des Oxidationsmittels von 10 ppm bis 5.000 ppm, bezogen auf die Gesamtmasse der in die Nitrierung zurückzuführenden aufkonzentrierten Schwefelsäure, einstellt.

Das Anfahren des kontinuierlichen Nitrobenzolprozesses wird in WO 2014/016292 A1 beschrieben, wobei während des Anfahrvorganges entweder ein Einsatzbenzol benutzt wird, das weniger als 1,5 % aliphatische organische Verbindungen, bezogen auf die Gesamtmenge des Einsatzbenzols, enthält oder nur Frischbenzol (das im Allgemeinen diesen Spezifikationsanforderungen genügt) eingesetzt wird.

WO 2014/016290 A1 beschreibt ebenfalls den Anfahrvorgang eines kontinuierlichen Nitrobenzolprozesses, wobei die Kreislaufschwefelsäure weniger als 1,0 % an organischen Verbindungen, insbesondere Nitrobenzol, bezogen auf die Gesamtmasse an Schwefelsäure im Schwefelsäure-Kreislauf, enthalten soll. Dies wird dadurch erreicht, dass man den am Ende eines oder vor einem neuen Produktionszyklus den Blitzverdampfer zur Aufkonzentrierung der Schwefelsäure bei einer erhöhten Temperatur betreibt, um auf diese Weise Nitrobenzol und Spuren von Benzol, Dinitrobenzol und Nitrophenol aus der Kreislaufschwefelsäure zu entfernen.

Daneben sind isotherme Verfahren zur Nitrierung von Benzol mit Mischsäure bekannt, wie beispielsweise in EP 0 156 199 B1 beschrieben.

Die Güte eines Reaktionsverfahrens zur Herstellung von Nitroaromaten ist also einerseits definiert durch den Gehalt des Roh-Produktes an unerwünschten Nebenkomponenten und Verunreinigungen, die durch unsachgemäße Reaktionsführung entstehen. Andererseits ist die Güte eines Reaktionsverfahrens dadurch definiert, dass der gesamte Prozess ohne technischen Produktionsausfall oder Problemen, die zu einem Eingriff in den Prozess nötigen, betrieben werden kann und dass Verluste an Einsatzstoffen vermieden oder zumindest minimal gehalten werden.

Großtechnische Produktionsanlagen sind für den Betrieb bei einer definierten Nenn-Produktionskapazität (auch als "Nennlast" bezeichnet) optimiert. Die Nenn-Produktionskapazität wird definiert durch den für eine gegebene Produktionsanlage maximal möglichen Durchsatz desjenigen Reaktanten, der die Ausbeute an gewünschtem Produkt bestimmt, also bei der Nitrierung von Benzol - da das Benzol im Überschuss eingesetzt wird - durch den maximal möglichen Durchsatz an Salpetersäure (auch als Salpetersäure-Last bezeichnet) unter den für die Produktionsanlage gewählten Randbedingungen (insbesondere Benzolüberschuss und Schwefelsäuremenge). Verringert man bspw. infolge gesunkener Nachfrage den Durchsatz an Salpetersäure - arbeitet man also im sog. Teillastbereich - so wird üblicherweise auch der Durchsatz der anderen Reaktanten entsprechend (also im gleichen Verhältnis) verringert. Wie die Betriebserfahrung lehrt, ist diese Vorgehensweise jedoch nicht immer frei von Problemen.

Allen oben aufgeführten Literaturstellen ist gemein, dass sie das Betreiben einer kontinuierlich betriebenen Nitrieranlage im Teillastbereich und die damit verbundenen Schwierigkeiten nicht beschreiben. Auch die oben erwähnten internationalen Anmeldungen WO 2014/016292 A1 und WO 2014/016290 A1 befassen sich nicht mit Lastwechseln *während des laufenden Produktionsbetriebs.* Beide Anmeldungen gehen nämlich vom Zustand einer *außer Betrieb befindlichen* Produktionsanlage aus (d. h. die Massenströme an Benzol, Salpeter- und Schwefelsäure sind im Ausgangszustand gleich null). Der Zeitraum von Beginn der kontinuierlichen Produktion (Anfahren) über die eigentliche kontinuierliche Produktion (möglichst bei Nennlast, erforderlichenfalls auch bei geringerer Last) bis zum Beenden der kontinuierlichen Produktion (Abfahren) wird auch als *"Produktionszyklus"* bezeichnet. Die beiden Patentanmeldungen WO 2014/016292 A1 und WO 2014/016290 A1 befassen sich mit dem ersten Teil eines solchen Produktionszyklus, dem Anfahren, während die vorliegende Erfindung mit dem mittleren Teil, der eigentlichen kontinuierlichen Produktion, befasst ist. Das in WO 2014/016292 A1 und WO 2014/016290 A1 beschriebene Anfahren hat zum Ziel, die Produktionsanlage möglichst rasch, dabei aber ohne Probleme, in den Zustand der angestrebten Soll-Last (Nennlast) zu bringen.

Laständerungen im laufenden Betrieb, also Lastreduzierungen insbesondere ausgehend von der Nennlast auf beispielsweise "Halblast" (= 50 % der Nennlast), sind nicht Gegenstand der Patentanmeldungen WO 2014/016292 A1 und WO 2014/016290 A1. Solche Laständerungen können aber Herausforderungen mit sich bringen, welche über die eines gewöhnlichen Anfahrvorgangs hinausgehen. Der Grund hierfür liegt letztlich darin, dass Produktionsanlagen *für den Betrieb bei Nennlast optimiert sind* und daher jede signifikante Abweichung vom Betrieb bei Nennlast dazu führt, dass eine Produktionsanlage unter wirtschaftlichen und/oder technischen Gesichtspunkten nur noch suboptimal läuft. So erhöht sich bei einer Verringerung der Salpetersäure-Last in einer Nitrieranlage auf einen Wert signifikant unterhalb der Nennlast auch die Verweilzeit in den Reaktionsapparaten signifikant, und zwar auf einen Wert, für den die Produktionsanlage nicht optimiert wurde. Dies kann mit vermehrter Nebenproduktbildung, unter Umständen verbunden mit vermehrter Bildung von Ablagerungen in den eingesetzten Apparaten, einhergehen.

Bei Lastwechseln im laufenden Produktionsbetrieb muss daher im Ausgangszustand oder im Endzustand, im Extremfall in beiden Zuständen, die Produktionsanlage für einen substanziellen Zeitraum - unter Umständen für Tage oder sogar Wochen, wenn etwa die Nachfrage im Markt temporär sinkt - unter Bedingungen betrieben werden, *für die sie nicht optimiert wurde.* Diesem Problem kann man nicht einfach mit strukturellen Anpassungen der Produktionsanlage begegnen. Die vorrichtungstechnischen Gegebenheiten einer Produktionsanlage stehen nämlich nach Fertigstellung derselben fest und sind nicht mehr - jedenfalls nicht ohne aufwändige Umbauten, die man selbstverständlich zu vermeiden sucht - veränderlich.

Weitere Verbesserungen der bekannten Verfahren zur Herstellung von Nitrobenzol wären daher wünschenswert. Insbesondere wäre es wünschenswert, die bekannten Verfahren zur Herstellung von Nitrobenzol so auszugestalten, dass diese auch in Teillastbereichen ohne Probleme, insbesondere was die Bildung unerwünschter Nebenprodukte oder gar Ablagerungen angeht, betrieben werden können. Weiterhin wäre es wünschenswert, die vollständige Stilllegung einzelner Produktionsstraßen bei reduzierter Nachfrage nach dem gewünschten Nitroaromaten möglichst vermeiden zu können, um mögliche Probleme beim An- und Abfahren sowie eventuelle Standschäden gar nicht erst aufkommen lassen zu können. Die Möglichkeit des Betriebs einer Produktionsanlage unter Teillastbedingungen unter Vermeidung oder zumindest Verringerung der oben genannten Probleme würde auch die Flexibilität erhöhen, indem etwa eine Produktionsanlage mit zwei Produktionsstraßen auch problemlos mit beispielsweise 60 % der Gesamt-Nennlast betrieben werden könnte und nicht nur mit 50 % (wie es bei vollständiger Stilllegung einer der beiden Produktionsstraßen und Betrieb der anderen Produktionsstraße bei Nennlast der Fall wäre).

Diesem Bedarf Rechnung tragend ist ein Gegenstand der vorliegenden Erfindung ein **kontinuierlich betrie-**

**benes Verfahren zur Herstellung von Nitrobenzol,** umfassend die Nitrierung von Benzol (1) mit Salpetersäure (2) und Schwefelsäure (3), bei dem

(i) der Nitrierung

- ein Benzol (1) enthaltender Strom 10 eines Massenanteils an Benzol (1) von $w_1$, wobei wi bevorzugt $\geq 0{,}950$, besonders bevorzugt $\geq 0{,}980$ ist, mit einem Massenstrom $\dot{m}_{10}$,

- ein Salpetersäure (2) enthaltender Strom 20 eines Massenanteils an Salpetersäure (2) von $w_2$, wobei $w_2$ bevorzugt im Bereich von 0,600 bis 0,750, besonders bevorzugt im Bereich von 0,650 bis 0,700 liegt, mit einem Massenstrom $\dot{m}_{20}$ und

- ein Schwefelsäure (3) enthaltender Strom 30 eines Massenanteils an Schwefelsäure von $w_3$, wobei $w_3$ bevorzugt im Bereich von 0,650 bis 0,750, besonders bevorzugt im Bereich von 0,690 bis 0,730 liegt, mit einem Massenstrom $\dot{m}_{30}$

zugeführt werden;

(ii) $\dot{m}_{10}$ und $\dot{m}_{20}$ bei gegebenen Werten für $w_1$ und $w_2$ stets so gewählt werden, dass Benzol (1) im stöchiometrischen Überschuss zu Salpetersäure (2) vorliegt, wobei

(iii) bei einer angestrebten Änderung der der Nitrierung über den Massenstrom $\dot{m}_{20}$ zugeführten Menge an Salpetersäure (2)

- von einem Ausgangszustand A definiert durch einen Massenstrom an Salpetersäure (2) $\dot{m}_2$ (A) = $\dot{m}_{20}$ (A) $\cdot$ $w_2$ (A), einem unter Beachtung von (ii) ausgewählten Massenstrom an Benzol (1) $\dot{m}_1$ (A) = $\dot{m}_{10}$ (A) $\cdot$ $w_1$ (A) und einem Massenstrom an Schwefelsäure (3) $\dot{m}_3$ (A) = $\dot{m}_{30}$ (A) $\cdot$ $w_3$ (A),
- in einen Endzustand E definiert durch einen Massenstrom an Salpetersäure (2) $\dot{m}_2$ (E) = $\dot{m}_{20}$ (E) $\cdot$ $w_2$ (E), einem unter Beachtung von (ii) ausgewählten Massenstrom an Benzol (1) $\dot{m}_1$ (E) = $\dot{m}_{10}$ (E) $\cdot$ $w_1$ (E) und einem Massenstrom an Schwefelsäure (3) $\dot{m}_3$ (E) = $\dot{m}_{30}$ (E) $\cdot$ $w_3$ (E),

der Massenstrom $\dot{m}_{20}$ und der Massenanteil $w_2$ so gewählt werden, dass sich der angestrebte Wert $\dot{m}_2$ (E) einstellt, wobei mindestens eine solche Änderung der der Nitrierung über den Massenstrom $\dot{m}_{20}$ zugeführten Menge an Salpetersäure (2) durchgeführt wird, und wobei diese mindestens eine Änderung der der Nitrierung über den Massenstrom $\dot{m}_{20}$ zugeführten Menge an Salpetersäure (2)

eine <u>Erniedrigung</u> auf einen Wert $\dot{m}_2$ (E) < $0{,}95 \cdot \dot{m}_2$ (A) für mehr als 0,50 Stunden ist, und wobei

das Verhältnis $\dot{m}_2$ (E) / $\dot{m}_3$ (E) im Vergleich zum Verhältnis $\dot{m}_2$ (A) / $\dot{m}_3$ (A) so erniedrigt wird, dass gilt:

$$0{,}45 \cdot \dot{m}_2\,(A) / \dot{m}_3\,(A) \leq \dot{m}_2\,(E) / \dot{m}_3\,(E) \leq 0{,}97 \cdot \dot{m}_2\,(A) / \dot{m}_3\,(A),$$

insbesondere

$$0{,}45 \cdot \dot{m}_2\,(A) / \dot{m}_3\,(A) \leq \dot{m}_2\,(E) / \dot{m}_3\,(E) \leq 0{,}95 \cdot \dot{m}_2\,(A) / \dot{m}_3\,(A)$$

und das Verhältnis $\dot{m}_1$ (E) / $\dot{m}_2$ (E) im Vergleich zum Verhältnis $\dot{m}_1$ (A) / $\dot{m}_2$ (A) maximal so geändert wird, dass gilt:

$$0{,}98 \cdot \dot{m}_1\,(A) / \dot{m}_2\,(A) \leq \dot{m}_1\,(E) / \dot{m}_2\,(E) \leq 1{,}02 \cdot \dot{m}_1\,(A) / \dot{m}_2\,(A).$$

Vollkommen überraschend wurde gefunden, dass es vorteilhaft ist, im Falle einer **Lasterniedrigung** *das Verhältnis $\dot{m}_2$ (E) / $\dot{m}_3$ (E) im Vergleich zum Verhältnis $\dot{m}_2$ (A) / $\dot{m}_3$ (A) signifikant zu erniedrigen*
Erfindungsgemäß werden *der Nitrierung die Ströme 10, 20 und 30 zugeführt.* Die Nitrierung wird in einem geeigneten Reaktor durchgeführt. Beispiele für geeignete Reaktoren werden weiter unten näher erläutert. Die drei genannten Ströme werden dem Reaktor der Nitrierung mit einem jeweiligen *Massenstrom $\dot{m}_i$* kontinuierlich zugeführt, angegeben z. B. in kg / h. Dabei werden $\dot{m}_{10}$ und $\dot{m}_{20}$ *bei gegebenen Werten für $w_1$ und $w_2$ stets so gewählt, dass Benzol (1) im stöchiometrischen Überschuss zu Salpetersäure (2)* vorliegt. Stöchiometrisch wird für die Einführung einer Nitrogruppe in ein Benzolmolekül ein Molekül Salpetersäure benötigt. Für die Nitrierung eines Mols Benzol zu Nitrobenzol bedarf es demnach stöchiometrisch eines Mols Salpetersäure. Den zugeführten Massenströmen an Einsatzmaterialien entsprechende Massenströme an Produkten (im Wesentlichen Nitrobenzol, überschüssiges Benzol und verdünnte, d. h. das Reaktionswasser enthaltende, Schwefelsäure) werden dem Reaktor der Nitrierung kontinuierlich entnommen.

Der Massenanteil $w_i$ einer Komponente i in einem Strom z bezeichnet den Quotienten aus dem Massenstrom der Komponente i und dem Gesamtmassenstrom des Stroms z. Beträgt beispielsweise der Massenanteil $w_1$ an Benzol 0,980, so besteht der *Benzol enthaltende Strom 10* zu 98,0 Massen-% aus Benzol und zu 2,0 Massen-% aus anderen Bestandteilen (z. B. aliphatische organische Verbindungen aus dem in bevorzugter Ausgestaltung vorhandenen Anteil an rezykliertem Benzol ("Rückbenzol") im Benzolstrom 10). Die *Massenanteile $w_2$ und $w_3$ des Salpetersäure- bzw. Schwefelsäurestroms 20 bzw. 30* bezeichnen den theoretischen Massenanteil an $HNO_3$ (2) bzw. $H_2SO_4$ (3) in dem jeweiligen Strom, ungeachtet der Tatsache, dass diese Säuren tatsächlich in dissoziierter Form vorliegen. Wird als Strom 30 bspw. eine 70,0%ige Schwefelsäure eingesetzt, so ist $w_3 = 0,700$.

Da Benzol (1) erfindungsgemäß im Überschuss eingesetzt wird, wird die Ausbeute an Nitrobenzol durch den Durchsatz (durch die *Last*) an eingesetzter Salpetersäure, also durch den Massenstrom $\dot{m}_2$, bestimmt. **Das erfindungsgemäße Verfahren umfasst mindestens eine Laständerung, d. h. eine *Änderung des Massenstroms $\dot{m}_2$ ausgehend von einem Ausgangszustand A mit $\dot{m}_2$ (A) in einen Endzustand E mit $\dot{m}_2$ (E).*** Unter einer *Änderung der der Nitrierung über den Massenstrom $\dot{m}_{20}$ zugeführten Menge an Salpetersäure (2)*, d. h. unter einer **Änderung des Massenstroms $\dot{m}_2$** in diesem Sinne ist eine Änderung um mehr als 5,00 % (d. h. es gilt $\dot{m}_2$ (E) < 0,95 · $\dot{m}_2$(A)) für einen Zeitraum von mehr als 0,50 Stunden, bevorzugt von mehr als 2,0 Stunden, besonders bevorzugt von mehr als 6,0 Stunden, ganz besonders bevorzugt vom mehr als 12 Stunden, außerordentlich ganz besonders bevorzugt von mehr als 24 Stunden, zu verstehen. Kleinere Änderungen oder Änderungen über einen kürzeren Zeitraum sind im Sinne dieser Erfindung als unbeabsichtigte Lastschwankungen, wie sie im betrieblichen Alltag immer wieder einmal vorkommen können, anzusehen. Bevorzugt bewegen sich Laständerungen im Sinne der vorliegenden Erfindung im wie folgt definierten Bereich: $0,40 · \dot{m}_2(A) \leq \dot{m}_2(E) < 0,95 · \dot{m}_2(A)$. Selbstverständlich kann das erfindungsgemäße Verfahren auch mehrere Laständerungen umfassen.

Beispielhaft sei folgend eine Lasthalbierung (d. h. $\dot{m}_2 (E) = 0,50 · \dot{m}_2(A)$) ausgehend von Produktion bei Nennlast betrachtet:

Im Betrieb bei Nennlast mit einem entsprechenden Massenstrom $\dot{m}_2 = \dot{m}_{2, Nenn}$ kann das erfindungsgemäße Verfahren durchgeführt werden wie aus dem Stand der Technik bekannt. Bei bekanntem $\dot{m}_2 = \dot{m}_{2, Nenn}$ werden $w_2$ (und damit $\dot{m}_{20}$) sowie die Parameter $w_i$ und $\dot{m}_i$ der Ströme 10 und 30 durch den Fachmann im Rahmen seines allgemeinen Fachwissens und unter Berücksichtigung der Randbedingungen der vorhandenen Produktionsanlage festgelegt. Die Produktionsanlage produziert so lange kontinuierlich das gewünschte Produkt, bis, z. B. aufgrund gesunkener Nachfrage, der Durchsatz an Salpetersäure (2) beispielsweise halbiert werden soll. In der Terminologie der vorliegenden Erfindung bedeutet dies eine Änderung der Betriebsweise ausgehend vom *Ausgangszustand A* (= Produktion mit Nennlast) in einen *Endzustand E* (Produktion mit Halblast). Mit der Lasterniedrigung einher geht zwangsläufig eine Halbierung des in die Nitrierung geführten Salpetersäuremassenstroms $\dot{m}_2$ Prinzipiell könnte diese Halbierung erreicht werden, indem $w_2$ halbiert und $\dot{m}_{20}$ konstant gehalten wird. Es ist jedoch bevorzugt, den Massenanteil $w_2$ bei der Laständerung gleich zu halten und $\dot{m}_{20}$ zu verändern (im konkreten Beispiel zu halbieren). Gleiches gilt jeweils für die Massenanteile $w_1$ und $w_3$. Im Unterschied zum Stand der Technik werden nun nicht einfach die Massenströme $\dot{m}_{10}$ und $\dot{m}_{30}$ im gleichen Maß reduziert wie $\dot{m}_{20}$, sondern es wird *das Verhältnis $\dot{m}_2$ (E) / $\dot{m}_3$ (E) im Vergleich zum Verhältnis $\dot{m}_2$ (A) / $\dot{m}_3$ (A) signifikant erniedrigt.* Dabei wird das Massenverhältnis $\dot{m}_1$ (E) / $\dot{m}_2$ (E) im Vergleich zu $\dot{m}_1$ (A) / $\dot{m}_2$ (A) im Wesentlichen gleich gehalten. Die Begriffe "signifikant erniedrigen" und "im Wesentlichen gleich halten" sind dabei durch die oben aufgeführten numerischen Grenzwerte definiert. Hält man, wie es erfindungsgemäß für alle Ausführungsformen bevorzugt ist, alle Massenanteile $w_i$ konstant, so ist diese Vorgehensweise gleichbedeutend mit der Aussage, dass *das Verhältnis $\dot{m}_{20}$ (E) / $\dot{m}_{30}$ (E) im Vergleich zum Verhältnis $\dot{m}_{20}$ (A) / $\dot{m}_{30}$ (A) signifikant erniedrigt* wird.

Es folgt zunächst eine Kurzzusammenfassung verschiedener möglicher Ausführungsformen der Erfindung:
In einer ersten Ausführungsform der Erfindung, die mit allen anderen Ausführungsformen kombiniert werden kann, wird die Nitrierung von Benzol zu Nitrobenzol adiabatisch betrieben.

In einer zweiten Ausführungsform der Erfindung, die eine besondere Ausgestaltung der ersten Ausführungsform ist, umfasst die Nitrierung von Benzol zu Nitrobenzol die folgenden Schritte:

(I) Nitrierung von Benzol mit Salpetersäure und Schwefelsäure unter Bildung von Nitrobenzol in einem Reaktor, wobei ein Benzol-haltiger Strom 10 mit einem Massenstrom $\dot{m}_{10}$, ein Salpetersäure-haltiger Strom 20 mit einem Massenstrom $\dot{m}_{20}$ und ein Schwefelsäure-haltiger Strom 30 mit einem Massenstrom $\dot{m}_{30}$ in den Reaktor eingetragen werden;

(II) Phasentrennung des Reaktionsgemisches aus Schritt (I) in einem Phasentrennapparat in eine wässrige, Schwefelsäure enthaltende Phase und eine organische, Nitrobenzol enthaltende Phase;

(III) Aufkonzentrierung der in Schritt (II) erhaltenen wässrigen Phase durch Verdampfung von Wasser in einem Verdampfungsapparat zu einer wässrigen Schwefelsäure-haltigen Phase mit erhöhter Schwefelsäurekonzentration, und wobei die aufkonzentrierte, Schwefelsäure-haltige, wässrige Phase in Schritt (I) zurückgeführt und als Bestandteil des Schwefelsäure-haltigen Stroms 30 eingesetzt wird;

(IV) mindestens zweistufiges Waschen der in Schritt (II) erhaltenen organischen, Nitrobenzol enthaltenden Phase und Abtrennung der wässrigen Phase nach jeder Stufe;

(V) Destillation, bevorzugt Rektifikation, der in der letzten Stufe von Schritt (IV) erhaltenen organischen, Nitrobenzol enthaltenden Phase unter Abtrennung von nicht umgesetztem Benzol, welches in Schritt (I) zurückgeführt und als Bestandteil des Benzol-haltigen Stroms 10 eingesetzt wird.

In einer **dritten Ausführungsform** der Erfindung, die eine besondere Ausgestaltung der zweiten Ausführungsform ist, schließt sich an Schritt (V) Folgendes an:

(VI) Aufarbeitung des Abwassers der ersten Waschstufe von Schritt (IV) umfassend Reinigung dieses Abwassers in einer Vorrichtung zur Destillation oder Strippung,

(VII) Aufarbeitung des Abwassers der zweiten Waschstufe von Schritt (IV) umfassend Reinigung dieses Abwassers in einer Vorrichtung zur Destillation oder Strippung, wobei der Vorrichtung zur Destillation oder Strippung eine Vorrichtung zur thermischen Druckzersetzung vor- und/oder nachgeschaltet sein kann.

In einer vierten Ausführungsform der Erfindung, die eine besondere Ausgestaltung der zweiten und dritten Ausführungsform ist, umfasst Schritt (IV) Folgendes:

(IVa) Waschen der in Schritt (II) erhaltenen organischen, Nitrobenzol enthaltenen Phase in mindestens einer Wäsche und anschließende Phasentrennung in eine wässrige und eine organische, Nitrobenzol enthaltende Phase (erste Waschstufe);

(IVb) Waschen der in Schritt (IVa) erhaltenen organischen Phase in mindestens einer alkalischen Wäsche mit einer wässrigen Lösung einer Base, die bevorzugt ausgewählt ist aus der Gruppe bestehend aus Natriumhydroxid, Natriumcarbonat und Natriumhydrogencarbonat, und anschließende Phasentrennung in eine wässrige und eine organische, Nitrobenzol enthaltende Phase (zweite Waschstufe);

(IVc) Waschen der in Schritt (IVb) erhaltenen organischen Phase in mindestens einer, bevorzugt zwei bis vier, besonders bevorzugt zwei bis drei, ganz besonders bevorzugt zwei neutralen Wäsche(n) mit Wasser und anschließender Phasentrennung in eine wässrige und eine organische, Nitrobenzol enthaltende organische Phase (dritte Waschstufe).

In einer **fünften Ausführungsform** der Erfindung, die mit allen anderen Ausführungsformen kombiniert werden kann, liegt das Verhältnis $\dot{m}_1$ (A) / $\dot{m}_2$ (A) im Bereich von 1,26 bis 1,74, bevorzugt im Bereich von 1,28 bis 1,61, besonders bevorzugt im Bereich von 1,29 bis 1,55 und ganz besonders bevorzugt im Bereich von 1,30 bis 1,49.

In einer sechsten Ausführungsform der Erfindung, die mit allen anderen Ausführungsformen kombiniert werden kann, erfolgt der Lastwechsel im wie folgt definierten Bereich:

$$0{,}40 \cdot \dot{m}_2\,(A) \leq \dot{m}_2\,(E) < 0{,}95 \cdot \dot{m}_2\,(A).$$

In einer **siebten Ausführungsform** der Erfindung, die eine besondere Ausgestaltung der sechsten Ausführungsform ist, gelten die folgenden Beziehungen:

wenn $\dot{m}_2$ (E) im Bereich von 0,80 · $\dot{m}_2$ (A) bis < 0,95 · $\dot{m}_2$ (A) liegt, wird $\dot{m}_2$ (E) / $\dot{m}_3$ (E) auf einen Wert im Bereich von 0,80 · $\dot{m}_2$ (A) / $\dot{m}_3$ (A) bis 0,97 · $\dot{m}_2$ (A) / $\dot{m}_3$ (A), insbesondere 0,80 · $\dot{m}_2$ (A) / $\dot{m}_3$ (A) bis 0,95 · $\dot{m}_2$ (A) / $\dot{m}_3$ (A) eingestellt;

wenn $\dot{m}_2$ (E) im Bereich von $0{,}65 \cdot \dot{m}_2$ (A) bis $< 0{,}80 \cdot \dot{m}_2$ (A) liegt, wird $\dot{m}_2$ (E) / $\dot{m}_3$ (E) auf einen Wert im Bereich von $0{,}65 \cdot \dot{m}_2$ (A) / $\dot{m}_3$ (A) bis $< 0{,}80 \cdot \dot{m}_2$ (A) / $\dot{m}_3$ (A) eingestellt; und

wenn $\dot{m}_2$ (E) im Bereich von $0{,}40 \cdot \dot{m}_2$ (A) bis $< 0{,}65 \cdot \dot{m}_2$ (A) liegt, wird $\dot{m}_2$ (E) / $\dot{m}_3$ (E) auf einen Wert im Bereich von $0{,}40 \cdot \dot{m}_2$ (A) / $\dot{m}_3$ (A) bis $< 0{,}65 \cdot \dot{m}_2$ (A) / $\dot{m}_3$ (A) eingestellt.

In einer achten Ausführungsform der Erfindung, die eine besondere Ausgestaltung der siebten Ausführungsform ist, gilt: $0{,}98 \cdot \dot{m}_3$ (A) $\leq \dot{m}_3$ (E) $\leq 1{,}02 \cdot \dot{m}_3$ (A), insbesondere $\dot{m}_3$ (A) $= \dot{m}_3$ (E).

In einer neunten Ausführungsform der Erfindung, die mit allen anderen Ausführungsformen kombiniert werden kann, gilt: $w_1$ (A) $= w_1$ (E).

In einer zehnten Ausführungsform der Erfindung, die mit allen anderen Ausführungsformen kombiniert werden kann, gilt: $w_2$ (A) $= w_2$ (E).

In einer elften Ausführungsform der Erfindung, die mit allen anderen Ausführungsformen kombiniert werden kann, gilt: $w_3$ (A) $= w_3$ (E).

In einer zwölften Ausführungsform der Erfindung, die mit allen anderen Ausführungsformen kombiniert werden kann, gilt: $w_1$ (A) $= w_1$ (E), $w_2$ (A) $= w_2$ (E) und $w_3$ (A) $= w_3$ (E).

In einer dreizehnten Ausführungsform der Erfindung, die mit allen anderen Ausführungsformen kombiniert werden kann, wird

das Verhältnis $\dot{m}_2$ (E) / $\dot{m}_3$ (E) im Vergleich zum Verhältnis $\dot{m}_2$ (A) / $\dot{m}_3$ (A) so erniedrigt, dass gilt:

$$0{,}45 \cdot \dot{m}_2\,(A) / \dot{m}_3\,(A) \leq \dot{m}_2\,(E) / \dot{m}_3\,(E) \leq 0{,}95 \cdot \dot{m}_2\,(A) / \dot{m}_3\,(A)$$

und das Verhältnis $\dot{m}_1$ (E) / $\dot{m}_2$ (E) im Vergleich zum Verhältnis $\dot{m}_1$ (A) / $\dot{m}_2$ (A) maximal so geändert, dass gilt:

$$0{,}98 \cdot \dot{m}_1\,(A) / \dot{m}_2\,(A) \leq \dot{m}_1\,(E) / \dot{m}_2\,(E) \leq 1{,}02 \cdot \dot{m}_1\,(A) / \dot{m}_2\,(A).$$

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

Der Benzol (1) enthaltende Strom 10 eines Massenanteils an Benzol (1) von $w_1$ besteht im Wesentlichen, d. h. bevorzugt zu mindestens 95,0 Massen-%, besonders bevorzugt zu mindestens 98,0 Massen-%, bezogen auf die Gesamtmasse des Stroms 10, aus Benzol, mit anderen Worten beträgt der Massenanteil an Benzol (1) im Strom 10, $w_1$, bevorzugt mindestens 0,950, besonders bevorzugt mindestens 0,980. Da Benzol erfindungsgemäß im Überschuss eingesetzt wird (vgl. (ii)), verbleibt immer ein Anteil an nicht umgesetztem Benzol, der bevorzugt, ggf. nach Aufreinigung, in die Nitrierung zurückgeführt und Bestandteil des Stroms 10 wird. Die in diesem rückgeführten Benzolstrom enthaltenden Verunreinigungen zusammen mit den im frisch zugeführten Benzol ggf. enthaltenden Verunreinigungen machen den übrigen Teil des Stroms 10 aus. Ebenso verbleibt nach der Reaktion Schwefelsäure, die lediglich verdünnt, aber nicht chemisch verbraucht ist. Bevorzugt wird diese Schwefelsäure aufkonzentriert und, ggf. nach Aufreinigung, in die Nitrierung zurückgeführt und als Bestandteil des Stroms 30 eingesetzt.

In einer Ausführungsform der vorliegenden Erfindung wird im Ausgangszustand im kontinuierlichen Betrieb bevorzugt ein stöchiometrischer Benzolüberschuss im Bereich von 2,00 % bis 40,0 %; besonders bevorzugt im Bereich von 3,00 % bis 30,0 %, ganz besonders bevorzugt im Bereich von 4,00 % bis 25,0 % und außerordentlich ganz besonders bevorzugt im Bereich von 5,00 % bis 20,00 % eingehalten. Dies entspricht einem Verhältnis $\dot{m}_1$ (A) / $\dot{m}_2$ (A) im Bereich von bevorzugt 1,26 bis 1,74, besonders bevorzugt 1,28 bis 1,61, ganz besonders bevorzugt 1,29 bis 1,55 und außerordentlich ganz besonders bevorzugt im Bereich von 1,30 bis 1,49. (Höhere Benzolüberschüsse als 20,00 % (wie etwa 45,00 %, 40,00 % oder 30,00 %) sind zwar im Allgemeinen nicht erstrebenswert, aber selbstverständlich möglich.) Diese Ausführungsform eignet sich bevorzugt für den Betrieb bei *Nennlast im Ausgangszustand* und *Teillast im Endzustand.* In jedem Fall ist es bevorzugt, den stöchiometrischen Benzolüberschuss so niedrig wie möglich zu halten (jedoch auch nicht zu niedrig, weil sonst vermehrt Dinitrobenzol entstehen kann). Hierdurch wird zum einen die Bildung unerwünschter Nebenprodukte (insbesondere feste Ablagerungen) verringert und zum anderen Energie eingespart, da weniger Benzol zurückgewonnen werden muss. Es ist daher insbesondere bevorzugt, im Ausgangszustand (insbesondere Produktion bei Nennlast) das Verhältnis $\dot{m}_1$ (A) / $\dot{m}_2$ (A) auf einen Wert im Bereich von 1,26 bis 1,49 (entsprechend einem stöchiometrischen Benzolüberschuss im Bereich von 2,00 % bis 20,00 %) einzustellen.

Wenn die Salpetersäure-Last ausgehend von einem Ausgangszustand mit einem bevorzugten stöchiometrischen Benzolüberschuss im Bereich von 2,00 % bis 40,0 %; besonders bevorzugt im Bereich von 3,00 % bis 30,0 %, ganz besonders bevorzugt im Bereich von 4,00 % bis 25,0 % und außerordentlich ganz besonders bevorzugt im Bereich von 5,00 % bis 20,00 % (entsprechend einem Verhältnis $\dot{m}_1$ (A) / $\dot{m}_2$ (A) im Bereich von bevorzugt 1,26 bis 1,74, besonders bevorzugt 1,28 bis 1,61, ganz besonders bevorzugt 1,29 bis 1,55 und außerordentlich ganz besonders bevorzugt im Bereich von 1,30 bis 1,49; siehe oben) erniedrigt werden soll, geschieht dies, in dem *das Verhältnis $\dot{m}_2$ (E) / $\dot{m}_3$ (E) im Vergleich zu $\dot{m}_2$ (A) / $\dot{m}_3$ (A)* erniedrigt wird. Bevorzugt gilt:

| Ausmaß des Lastwechsels: $\dot{m}_2$(E) liegt im Bereich | Ausmaß der Erniedrigung: $\dot{m}_2$ (E) / $\dot{m}_3$ (E) liegt im Bereich |
|---|---|
| von 0,80 · $\dot{m}_2$ (A) bis < 0,95 · $\dot{m}_2$ (A) | von 0,80 · $\dot{m}_2$ (A) / $\dot{m}_3$ (A) bis 0,97 · $\dot{m}_2$ (A) / $\dot{m}_3$ (A). insbesondere 0,80 · $\dot{m}_2$ (A) / $\dot{m}_3$ (A) bis 0,95 · $\dot{m}_2$ (A) / $\dot{m}_3$ (A) |
| von 0,65 · $\dot{m}_2$ (A) bis < 0,80 · $\dot{m}_2$ (A) | von 0,65 · $\dot{m}_2$ (A) / $\dot{m}_3$ (A) bis < 0,80 · $\dot{m}_2$ (A) / $\dot{m}_3$ (A) |
| von 0,40 · $\dot{m}_2$ (A) bis < 0,65 · $\dot{m}_2$ (A) | von 0,40 · $\dot{m}_2$ (A) / $\dot{m}_3$ (A) bis < 0,65 · $\dot{m}_2$ (A) / $\dot{m}_3$ (A) |

Es ist besonders bevorzugt, den Absolutwert $\dot{m}_3$ konstant oder zumindest im Wesentlichen ($\pm$ 2,0 %) konstant zu halten (*d. h. es gilt besonders bevorzugt 0,98 · $\dot{m}_3$ (A) $\leq \dot{m}_3$ (E) $\leq$ 1,02 · $\dot{m}_3$ (A)*). Wird, wie bevorzugt, auch $w_3$ konstant gehalten, bedeutet dies, dass der Massenstrom $\dot{m}_{30}$ bevorzugt beibehalten oder im Wesentlichen ($\pm$ 2,0 %) beibehalten wird. Wird $\dot{m}_3$ gleichgehalten, so entspricht die prozentuale Lasterniedrigung der prozentualen Erniedrigung des Verhältnisses $\dot{m}_2$ / $\dot{m}_3$. Bei einer Lasterniedrigung auf beispielsweise 75 % des Ausgangswerts (d. h. $\dot{m}_2$ (E) = 0,75 · $\dot{m}_2$ (A)) wird dann das Verhältnis $\dot{m}_2$ / $\dot{m}_3$ ebenfalls auf 75 % des Ausgangswerts erniedrigt (d. h. $\dot{m}_2$ (E) / $\dot{m}_3$ (E) = 0,75 · $\dot{m}_2$ (A) / $\dot{m}_3$ (A)).

Die Nitrierung von Benzol zu Nitrobenzol wird bevorzugt adiabatisch betrieben und umfasst besonders bevorzugt die folgenden Schritte:

(I) Nitrierung von Benzol mit Salpetersäure und Schwefelsäure unter Bildung von Nitrobenzol in einem Reaktor, wobei ein Benzol-haltiger Strom 10 mit einem Massenstrom $\dot{m}_{10}$, ein Salpetersäure-haltiger Strom 20 mit einem Massenstrom $\dot{m}_{20}$ und Schwefelsäure-haltiger Strom 30 mit einem Massenstrom $\dot{m}_3$ in den Reaktor eingetragen werden;

(II) Phasentrennung des Reaktionsgemisches aus Schritt (I) in einem Phasentrennapparat in eine wässrige, Schwefelsäure enthaltende Phase und eine organische, Nitrobenzol enthaltende Phase;

(III) Aufkonzentrierung der in Schritt (II) erhaltenen wässrigen Phase durch Verdampfung von Wasser in einem Verdampfungsapparat (dem sog. "Blitzverdampfer") zu einer wässrigen Schwefelsäure-haltigen Phase mit erhöhter Schwefelsäurekonzentration, und wobei die aufkonzentrierte, Schwefelsäure-haltige, wässrige Phase in Schritt (I) zurückgeführt und als Bestandteil des Schwefelsäure-haltigen Stroms 30 eingesetzt wird;

(IV) mindestens zweistufiges Waschen der in Schritt (II) erhaltenen organischen, Nitrobenzol enthaltenden Phase und Abtrennung der wässrigen Phase nach jeder Stufe;

(V) Destillation, bevorzugt Rektifikation, der in der letzten Stufe von Schritt (IV) erhaltenen organischen, Nitrobenzol enthaltenden Phase unter Abtrennung von nicht umgesetztem Benzol, welches in Schritt (I) zurückgeführt und als Bestandteil des Benzol-haltigen Stroms 10 eingesetzt wird,

(VI) optional und bevorzugt, Aufarbeitung des Abwassers der ersten Waschstufe von Schritt (IV) umfassend Reinigung dieses Abwassers in einer Vorrichtung zur Destillation oder Strippung,

(VII) optional und bevorzugt, Aufarbeitung des Abwassers der zweiten Waschstufe von Schritt (IV) umfassend Reinigung dieses Abwassers in einer Vorrichtung zur Destillation oder Strippung, wobei der Vorrichtung zur Destillation oder Strippung eine Vorrichtung zur thermischen Druckzersetzung vor- und/oder nachgeschaltet sein kann.

**Schritt (I)** kann grundsätzlich nach allen aus dem Stand der Technik bekannten adiabatisch betriebenen Nitrierverfahren durchgeführt werden. Für die Ausführung dieses Schritts des erfindungsgemäßen Verfahrens wird bevorzugt ein Rohrreaktor eingesetzt, in dem mehrere Dispergierelemente über die Länge des Reaktors verteilt angeordnet sind, die eine intensive Dispergierung und Durchmischung von Benzol, Salpetersäure und Schwefelsäure gewährleisten.

Ein solcher Reaktor, sowie die Form einsetzbarer Dispergierelemente, sind beispielsweise in EP 0708 076 B1 (FIG. 2) und EP 1 291 078 A2 (FIG. 1) beschrieben. Bevorzugt wird Schritt (I) in einer Verfahrensführung ausgeführt, wie sie in DE 10 2008 048 713 A1, insbesondere Absatz [0024], beschrieben ist.

Die Phasentrennung in **Schritt (II)** erfolgt ebenfalls nach aus dem Stand der Technik an sich bekannten Verfahren in einem dem Fachmann bekannten Trennbehälter. Die wässrige Phase enthält im Wesentlichen (infolge der Bildung von Reaktionswasser verdünnte) Schwefelsäure neben anorganischen Verunreinigungen, die organische Phase enthält im Wesentlichen Nitrobenzol neben überschüssigem Benzol und organischen Verunreinigungen.

Die Aufkonzentrierung der wässrigen Phase in **Schritt (III)** erfolgt grundsätzlich wie aus dem Stand der Technik bekannt. Die Schwefelsäure in der wässrigen Phase wird in einem Entspannungsverdampfer (auch als Blitzverdampfer bezeichnet) aufkonzentriert, indem Wasser in einen Bereich reduzierten Druckes hinein verdampft wird. Bei richtiger Wahl der Reaktionsbedingungen in der adiabatisch durchgeführten Nitrierung von Benzol mit Mischsäure erzielt man mit der Reaktionswärme der exothermen Reaktion eine so starke Erhitzung der Schwefelsäure enthaltenden wässrigen Phase, dass im Entspannungsverdampfer gleichzeitig wieder die Konzentration und Temperatur der Schwefelsäure enthaltenden wässrigen Phase eingestellt werden kann, die diese vor der Reaktion mit Benzol und Salpetersäure bei Eintritt in den Reaktorraum hatte. Dies ist beispielsweise beschrieben in EP 2 354 117 A1, insbesondere Absatz [0045]. Die so erhaltene aufkonzentrierte Schwefelsäure wird rezykliert und als Bestandteil des Stroms 30 eingesetzt.

Bevorzugt umfasst der **Schritt (IV)** die Schritte:

(IVa) Waschen der in Schritt (II) erhaltenen organischen, Nitrobenzol enthaltenen Phase in mindestens einer Wäsche und anschließende Phasentrennung in eine wässrige und eine organische, Nitrobenzol enthaltende Phase (erste Waschstufe). Die organische Phase, die üblicherweise noch Spuren an Säure enthält, wird dabei bevorzugt in einer bis zwei, bevorzugt einer Wäsche(n) mit einer wässrigen Waschflüssigkeit gewaschen und dann von der sauren wässrigen Phase durch Phasentrennung getrennt (bei mehreren Wäschen nach jeder einzelnen Wäsche). Bei diesem Vorgang werden die Säurereste, welche das rohe Nitrobenzol enthält, ausgewaschen; daher wird dieser Verfahrensschritt auch als *saure Wäsche* bezeichnet. Bevorzugt wird dabei so verfahren, dass sich in der nach der Phasentrennung der sauren Wäsche erhaltenen wässrigen Phase ein pH Wert < 5 (gemessen bei 20 °C) eingestellt. Als wässrige Waschflüssigkeit kann in dabei jede Art von Wasser, z. B. vollentsalztes Wasser oder Dampfkondensat, eingesetzt werden. Das Wasser kann auch gelöste Salze enthalten. Bevorzugt werden zur Durchführung dieser sauren Wäsche im Betrieb anfallende wässrige Ströme rezyklisiert.

(IVb) Waschen der in Schritt (IVa) erhaltenen organischen Phase in mindestens einer alkalischen Wäsche mit einer wässrigen Lösung einer Base, die bevorzugt ausgewählt ist aus der Gruppe bestehend aus Natriumhydroxid, Natriumcarbonat und Natriumhydrogencarbonat, und anschließende Phasentrennung in eine wässrige und eine organische, Nitrobenzol enthaltende Phase (zweite Waschstufe). Besonders bevorzugt wird als wässrige Baselösung Natronlauge eingesetzt. Die alkalische Wäsche wird im Folgenden anhand von Natronlauge beschrieben; für den Fachmann ist es ein Leichtes, bei Einsatz anderer Basen erforderlichenfalls entsprechende Abänderungen vorzunehmen. Bevorzugt hat die eingesetzte Natronlauge einen pH-Wert von 9,0 bis 14 (gemessen bei 20 °C). Das Massenverhältnis von Natronlauge zu organischer Phase (im Wesentlichen Nitrobenzol) ist abhängig vom in Schritt (I) eingesetzten Benzolüberschuss und beträgt bevorzugt 1 : 80 bis 1 : 500. Der pH-Wert der eingesetzten Natronlauge und ihr Massenverhältnis zur organischen Phase werden so eingestellt, dass acide Verunreinigungen (z. B. als Nebenprodukte gebildete Nitrophenole und in Schritt (II) nicht vollständig entfernte Säurereste) in dieser *alkalischen Wäsche* weitgehend bis vollständig, bevorzugt vollständig, neutralisiert werden. Die anschließende Aufarbeitung des alkalischen Abwassers kann nach den Verfahren des Standes der Technik, z. B. gemäß EP 1 593 654 A1 und EP 1 132 347 A2 erfolgen. Die so erhaltene organische, Nitrobenzol enthaltende Phase weist bevorzugt eine Temperatur von 20 °C bis 60 °C, besonders bevorzugt von 30 °C bis 50 °C auf. Bevorzugt enthält sie neben Nitrobenzol 4,0 bis 10 Massen % Benzol, und weniger als 100 ppm, besonders bevorzugt weniger als 60 ppm, an Nitrophenolen, jeweils bezogen auf die Gesamtmasse der erhaltenen organischen Phase.

(IVc) Waschen der in Schritt (IVb) erhaltenen organischen Phase in mindestens einer, bevorzugt zwei bis vier, besonders bevorzugt zwei bis drei, ganz besonders bevorzugt zwei neutralen Wäsche(n) mit Wasser und anschließender Phasentrennung in eine wässrige und eine organische, Nitrobenzol enthaltende organische Phase (dritte Waschstufe). Dies kann grundsätzlich nach allen im Stand der Technik üblichen Verfahren geschehen. Als Waschwasser wird dabei bevorzugt vollentsalztes Wasser (VE-Wasser), besonders bevorzugt eine Mischung aus VE-Wasser und Dampfkondensat (d. i. ein Kondensat von Wasserdampf, welcher durch Wärmeaustausch von Wasser mit beliebigen exothermen Verfahrensschritten erhalten wurde) und ganz besonders bevorzugt Dampfkondensat eingesetzt. Bevorzugt ist eine Verfahrensweise, bei der in der letzten neutralen Wäsche eine Elektrophorese eingesetzt wird (siehe WO 2012/013678 A2).

Das gewaschene Nitrobenzol wird schließlich in **Schritt (V)** von gelöstem Wasser, nicht umgesetztem Benzol und ggf. organischen Verunreinigungen durch weitere Aufarbeitung befreit. Diese Aufarbeitung erfolgt durch Destillation, insbesondere durch Rektifikation, wobei über Kopf die Brüden Wasser und Benzol und ggf. organische Verunreinigungen ausgetrieben werden. Die Brüden werden gekühlt und in einen Trennbehälter gefahren. In der unteren Phase setzt sich Wasser ab, das abgetrennt wird. In der oberen Phase befinden sich Benzol und Leichtsieder. Diese obere Phase wird, ggf. nach weiterer Aufreinigung, als Rückbenzol wieder der Nitrierung in Schritt (I) zugeführt und ist dort Bestandteil des Stroms 10. Bevorzugt wird als Destillationsapparat eine Rektifizierkolonne eingesetzt. Das Sumpfprodukt der Destillation wird, ggf. nach einer weiteren Destillation, in welcher Nitrobenzol als Destillat (also als Kopf- oder Seitenstromprodukt) erhalten wird, als Rein-Nitrobenzol weiteren Anwendungen (wie der Hydrierung zu Anilin) zugeführt.

Die saure wässrige Phase der Wäsche aus Schritt (IVa) wird bevorzugt in einem **Schritt (VI)** in einer sauren Abwasseraufarbeitung von Organika befreit und in eine biologische Kläranlage gefahren. Die saure Abwasseraufarbeitung umfasst insbesondere einen Abwasservorlagetank, einen Wärmeaustauscher, eine Abwasserdestillation mit Kondensationssystem, einen Abwasserkühler und einen Ablauf zur sauren Wäsche. Die nicht kondensierten Brüden der Abwasserdestillation, enthaltend Benzol und Nitrobenzol, werden in die Wäsche gemäß Schritt (IV), insbesondere in die saure Wäsche gemäß Schritt (IVa), rezykliert.

Die alkalische wässrige Phase der Wäsche aus Schritt (IVb) wird bevorzugt in einem **Schritt (VII)** in einer alkalischen Abwasseraufarbeitung, umfassend einen Abwasservorlagetank, einen Wärmeaustauscher, eine Abwasserdestillation mit Kondensationssystem und eine Anlage zur thermischen Druckzersetzung (TDZ), von Organika befreit. Unter *thermischer Druckzersetzung* wird im Rahmen der vorliegenden Erfindung ein Verfahren zur Aufarbeitung alkalischen Abwassers verstanden, bei dem organische Verunreinigungen unter Einwirkung von erhöhtem Druck und erhöhter Temperatur zersetzt werden. Geeignete Verfahren sind dem Fachmann bekannt und beispielsweise in EP 1 593 654 B1 beschrieben. Im Rahmen der vorliegenden Erfindung ist es insbesondere bevorzugt, das (ggf. in der Vorrichtung zur Destillation oder Strippung vorbehandelte) alkalische Abwasser unter Ausschluss von Sauerstoff auf Temperaturen von 150 °C bis 500 °C unter einem absoluten Druck von 50 bar bis 350 bar zu erhitzen.

Trotz erhöhter Mengen an Aromat und/oder erhöhter Mengen an Schwefelsäure hat sich überraschenderweise herausgestellt, dass die erfindungsgemäße Vorgehensweise eine Reihe von Vorteilen mit sich bringt, die das erfindungsgemäße Verfahren trotzdem attraktiv machen:

i) Die Bildung von Nebenprodukten wie Pikrinsäure oder Stickoxiden ($NO_x$) wird reduziert.

ii) Die Schwefelsäureverluste in der sauren Wäsche fallen geringer aus.

iii) Der große Überschuss an Benzol führt zu einer besseren Phasentrennung nach der Nitrierung und nach den Wäschen. Dies gilt insbesondere für die sog. saure Wäsche (Verringerung der Gefahr der Bildung einer stabilen Emulsion und damit Unmöglichkeit der Phasentrennung).

iv) Das erfindungsgemäße Verfahren bietet eine sinnvolle Alternative zur Stilllegung einzelner parallel betriebener Nitrierstraßen bei gesunkener Nachfrage. Anstatt bspw. bei zwei vorhandenen Nitrierstraßen eine davon bei Nennlast zu betreiben und die andere vollständig stillzulegen, können mit dem erfindungsgemäßen Verfahren beide Straßen bei Halblast betrieben werden, wodurch ein teures, energieintensives Wiederanfahren der zweiten Nitrierstraße bei wieder gestiegenem Bedarf vermieden werden kann. Dadurch werden auch alle Apparateteile (bspw. Pumpen), die nicht abgestellt und nach einiger Zeit erneut in Betrieb genommen werden müssen, geschont.

v) Durch die gute Produktqualität des Rein-Nitrobenzols auch bei Teillastbetrieb ergeben sich für den Einsatz eines solchen Nitrobenzols in weiteren Anwendungen, insbesondere in einem katalytischen Gasphasen-Anilinverfahren, Vorteile. Zusätzliche Verunreinigungen der Nitrobenzol-Erhitzer und -Verdampfer werden vermieden. Im Falle einer Eindüsung des Nitrobenzols in einen Wasserstoffgasstrom wird auch eine zusätzliche Verunreinigung der Katalysatoroberfläche, die zu einer Verringerung der Selektivität und/oder der Lebensdauer des Katalysators führt, vermieden.

### Beispiele

Gehaltsangaben in ppm oder % sind Massenanteile bezogen auf die Gesamtmasse des jeweiligen Stoff(strom)s. Analysenwerte wurden, sofern nicht anders angegeben, mittels Hochleistungsflüssigkeitschromatographie (HPLC - Nitrophenole) und Gaschromatographie (GC - andere Nebenprodukte und Benzol) und bestimmt.

### A. Allgemeine Bedingungen für die Herstellung von Nitrobenzol im Regelbetrieb bei Nennlast

In einen Nitrierreaktor werden ein Schwefelsäurestrom ($\dot{m}_{30}$ = 210 t/h; $w_3$ = 0,713), ein Salpetersäurestrom ($\dot{m}_{20}$ = 10000 kg/h; $w_2$ = 0,685) und ein Benzolstrom ($\dot{m}_{10}$ = 9 800 kg/h; $w_1$ = 0,989) bestehend aus 95 Massen-% Frischbenzol und 5 Massen-% Rückbenzol eindosiert. Es wird ein 14,14%iger Überschuss an Benzol, bezogen auf Salpetersäure, eingesetzt. Nach vollständiger Umsetzung der Salpetersäure mit dem Benzol zu Nitrobenzol unter adiabatischer Reaktionsführung wird das nunmehr ca. 130 °C heiße Reaktionsprodukt einem Phasentrennapparat zugeführt, in dem das Reaktionsprodukt in eine organische Phase (= Roh-Nitrobenzol, enthaltend neben Nitrobenzol noch Benzol) und eine wässrige Phase (= Absäure, enthaltend neben Schwefelsäure noch geringe Anteile an Nitrobenzol und Benzol) zerfällt. Die wässrige, hauptsächlich Schwefelsäure umfassende Phase wird im Verdampfer durch plötzliche Druckerniedrigung einer Blitzverdampfung von Wasser unterzogen und auf diese Weise aufkonzentriert. Die aufkonzentrierte Schwefelsäure wird im Schwefelsäuretank zur erneuten Verwendung gelagert. Nach dem Abtrennen im Phasentrennapparat wird das Rohnitrobenzol in der Rohnitrobenzolkühlung auf ca. 50 °C abgekühlt und der Wäsche zugeführt. Diese Wäsche umfasst eine saure, eine alkalische und eine neutrale Waschstufe.

Der so erhaltene von Nitrophenolen und Salzen weitgehend befreite Strom gereinigten Roh-Nitrobenzols wird wieder aufgeheizt und in einer Destillationskolonne von Wasser, Benzol und anderen Leichtsiedern, welche über Kopf abgetrennt werden, befreit, wodurch getrocknetes Rein-Nitrobenzol erhalten wird. Das kondensierte Kopfprodukt der Destillationskolonne wird einem Phasentrennapparat zugeführt, in dem das Kopfprodukt in eine organische Phase (enthaltend Benzol) und eine wässrige Phase zerfällt. Die organische Phase wird in einem Puffertank zwischengelagert und von dort, wie oben schon beschrieben, zur Reaktion in den Zulauf des Nitrierreaktors zurückgefahren.

Das in der alkalischen Wäsche anfallende Abwasser wird wie folgt aufgearbeitet:

Das Abwasser aus der alkalischen Wäsche wird in einen Absetztank gefahren, in dem ungelöstes Benzol und Nitrobenzol abgeschieden werden. 3,5 Tonnen pro Stunde an alkalischem Abwasser, das im Mittel einen Gehalt an Nitrobenzol von 2870 ppm, an Benzol von 409 ppm und an Nitrophenolen von 11809 ppm sowie einen pH-Wert von 12,8 hat (1,8 % NaOH-Überschuss gegenüber dem Ausgangsgehalt an Nitrophenolen vor der alkalischen Wäsche) wird in eine Strippkolonne gefahren, um aus diesem alkalischen Abwasser Benzol und Nitrobenzol durch Strippen mit Wasserdampf über Kopf zu entfernen. Dafür werden 500 kg/h 6-bar-Dampf verwendet. Der Druck im Kolonnenkopf beträgt 1,05 bar (absolut), und die Temperatur liegt bei 99,5 °C. Der Kopf der Strippkolonne ist mit einem vertikalen Kondensator bestückt, in dem die Benzol und Nitrobenzol enthaltenden Brüden auskondensiert und anschließend in die saure Wäsche zurückgeführt werden. Das feuchte, 99 °C heiße Abgas der Strippkolonne wird direkt in den Kondensator geführt und mit 30 °C warmen, sauren Wasser aus dem Sauerwassertank abgespritzt. Dadurch wird die mögliche Ablagerung von Ammoniumnitrat und/oder Ammoniumnitrit, die bei der Verwendung einer konventionellen Abgasleitung für die separate Ableitung des Abgases aus dem Kondensator im trockenen Bereich einer solchen Abgasleitung entstehen können, verhindert (die genannten Ammoniumsalze können aus in dem alkalischen Abwasser befindlichen Ammoniak und Stickoxiden entstehen). Das saure Wasser wird zusammen mit den auskondensierten Brüden der sauren Wäsche zugeführt. Eine Fehlfunktion der Strippkolonne kann beispielsweise durch redundante Sicherheitseinrichtungen überwacht werden. Nach der Strippung wird ein alkalisches Abwasser erhalten, das Benzol nur noch in einer Konzentration von bis zu 10 ppm und Nitrobenzol in einer Konzentration von bis zu 10 ppm enthält. Anschließend wird das so behandelte alkalische Abwasser in einer Anlage zur thermischen Druckzersetzung bei einer Verweilzeit von 20 min, einer Temperatur von 290 °C und einem absoluten Druck von 90 bar behandelt. Das dabei entstandene Abwasser wird auf 80 °C abgekühlt. Danach wird das Abwasser mit Direktdampf gestrippt. Im Sumpf der Strippkolonne wird ein Strom von 4,0 Tonnen pro Stunde bei einem absoluten Druck von 1,02 bar erhalten, der im Wesentlichen Wasser, Ammoniak, Kohlendioxid und Organika enthält. Das Kopfprodukt wird kondensiert und auf 35 °C abgekühlt. Aus dem Kondensat wird ein Purge-Strom an Organika ausgeschleust. 0,25 Tonnen pro Stunde des an Organika abgereicherten wässrigen Kondensat-Stromes werden als Rückfluss in die Strippkolonne zurückgeführt. Der Anteil an Organika im erhaltenen Abwasser, das einer biologischen Kläranlage zugeführt wird, beträgt 4726 ppm. Der Gehalt an Ammonium im Abwasser ist kleiner als 87 ppm. Es gibt im Regelfall keinerlei Probleme mit Ablagerungen im Bereich des Abgases der Strippkolonne.

Auf diese Weise hergestelltes Nitrobenzol hat im Mittel eine Reinheit von ca. 99,96 % (GC), einen Restbenzolgehalt von 0,0028 % (GC) und einen Wassergehalt von 0,0079 % (bestimmt nach Karl-Fischer). Die folgende Tabelle stellt die Betriebsbedingungen bei Nennlast zusammen.

**Tabelle 1:** Betriebsbedingungen bei Nennlast

| $\dot{m}_{10}$ / (kg h$^{-1}$) | $w_1$ | $\dot{m}_{20}$ / (kg h$^{-1}$) | $w_2$ | $\dot{m}_1$ / $\dot{m}_2$ | $\dot{m}_{30}$ / (t · h$^{-1}$) | $w_3$ | $\dot{m}_2$ / $\dot{m}_3$ |
|---|---|---|---|---|---|---|---|
| 9 800 | 0,989 | 10 000 | 0,685 | 1,41 | 210 | 0,713 | 0,0457 |

**Anmerkungen:**

$$\dot{m}_1 / \dot{m}_2 = \dot{m}_{10} / \dot{m}_{20} \cdot w_1/w_2; \dot{m}_2 / \dot{m}_3 = \dot{m}_{20} / \dot{m}_{30} \cdot w_2/w_3.$$

## B. Herstellung von Nitrobenzol bei geringer als Nennlast

Alle Massenanteile $w_i$ wurden gegenüber der Betriebsweise bei Nennlast gleichgehalten.

**Beispiel 1 (Vergleich): Verringerung der Salpetersäure-Last auf ca. 93 % der Nennlast unter entsprechender Verringerung von $\dot{m}_{10}$ und entsprechender Verringerung von $\dot{m}_{30}$**

Ausgehend von der Produktion bei Nennlast, wie zuvor unter A beschrieben, wurde der Massenstrom $\dot{m}_{20}$ auf 9301 kg/h (entsprechend $\dot{m}_{20}$ (E) / $\dot{m}_{20}$ (A) = $\dot{m}_2$ (E) / $\dot{m}_2$ (A) = 0,9301) reduziert. Der Massenstrom $\dot{m}_{10}$ wurde auf 8 954 kg/h reduziert, d. h. das Verhältnis $\dot{m}_1$ (E) / $\dot{m}_2$ (E) wurde mit 1,39 im Vergleich zum Ausgangszustand im Wesentlichen beibehalten.

Der Massenstrom $\dot{m}_{30}$ wurde auf 195 t/h reduziert, d. h. das Verhältnis $\dot{m}_2$ / $\dot{m}_3$ wurde mit 0,0458 im Wesentlichen beibehalten.

**Beispiel 2 (erfindungsgemäß): Verringerung der Salpetersäure-Last auf ca. 93 % der Nennlast unter Verringerung von $\dot{m}_{10}$ und Beibehaltung von $\dot{m}_{30}$**

Ausgehend von der Produktion bei Nennlast wie zuvor unter A beschrieben wurde der Massenstrom $\dot{m}_{20}$ auf 9298 kg/h (entsprechend $\dot{m}_{20}$ (E) / $\dot{m}_{20}$ (A) = $\dot{m}_2$ (E) / $\dot{m}_2$ (A) = 0,9298) reduziert. Der Massenstrom $\dot{m}_{10}$ wurde auf 8 937 kg/h reduziert, d. h. das Verhältnis $\dot{m}_1$ (E) / $\dot{m}_2$ (E) wurde mit 1,39 im Vergleich zum Ausgangszustand im Wesentlichen beibehalten. Die geringfügigen nominellen Unterschiede bei $\dot{m}_{10}$ (E) und $\dot{m}_{20}$ (E) im Vergleich zu Beispiel 1 beinträchtigen die Vergleichbarkeit nicht.

Der Massenstrom $\dot{m}_{30}$ wurde konstant gehalten, d. h. das Verhältnis $\dot{m}_2$ / $\dot{m}_3$ wurde auf 0,0425 *erniedrigt*.

**Beispiel 3 (Vergleich): Verringerung der Salpetersäure-Last auf ca. 60 % der Nennlast unter entsprechender Verringerung von $\dot{m}_{10}$ und entsprechender Verringerung von $\dot{m}_{30}$**

Ausgehend von der Produktion bei Nennlast, wie zuvor unter A beschrieben, wurde der Massenstrom $\dot{m}_{20}$ auf 6052 kg/h (entsprechend $\dot{m}_{20}$ (E) / $\dot{m}_{20}$ (A) = $\dot{m}_2$ (E) / $\dot{m}_2$ (A) = 0,6052) reduziert. Der Massenstrom $\dot{m}_{10}$ wurde auf 5 956 kg/h reduziert, d. h. das Verhältnis $\dot{m}_1$ (E) / $\dot{m}_2$ (E) wurde mit 1,42 im Vergleich zum Ausgangszustand im Wesentlichen beibehalten.

Der Massenstrom $\dot{m}_{30}$ wurde auf 127 t/h reduziert, d. h. das Verhältnis $\dot{m}_2$ / $\dot{m}_3$ wurde mit 0,0458 im Vergleich zum Ausgangszustand im Wesentlichen konstant gehalten.

**Beispiel 4 (erfindungsgemäß): Verringerung der Salpetersäure-Last auf ca. 60 % der Nennlast unter entsprechender Verringerung von $\dot{m}_{10}$ und (im Wesentlichen) Beibehaltung von $\dot{m}_{30}$**

Ausgehend von der Produktion bei Nennlast wie zuvor unter A beschrieben wurde der Massenstrom $\dot{m}_{20}$ auf 6005 kg/h (entsprechend $\dot{m}_{20}$ (E) / $\dot{m}_{20}$ (A) = $\dot{m}_2$ (E) / $\dot{m}_2$ (A) = 0,6005) reduziert. Der Massenstrom $\dot{m}_{10}$ wurde auf 5 945 kg/h reduziert, d. h. das Verhältnis $\dot{m}_1$ (E) / $\dot{m}_2$ (E) wurde mit 1,43 im Vergleich zum Ausgangszustand im Wesentlichen beibehalten. Die geringfügigen nominellen Unterschiede bei $\dot{m}_{10}$ (E) und $\dot{m}_{20}$ (E) im Vergleich zu Beispiel 3 beinträchtigen die Vergleichbarkeit nicht.

Der Massenstrom $\dot{m}_{30}$ wurde nur minimal auf 207 t/h reduziert, d. h. das Verhältnis $\dot{m}_2$ / $\dot{m}_3$ wurde im Vergleich zum Ausgangszustand auf 0,0279 *erniedrigt.*

Die folgende Tabelle stellt die Nebenproduktgehalte einander gegenüber.

**Tabelle 2: Nebenproduktgehalte des Nitrobenzols**

| Nitrobenzol aus | $\dot{m}_1$ / $\dot{m}_2$ *im Vergleich zu A* | $\dot{m}_2$ / $\dot{m}_3$ *im Vergleich Zu A* | $C_{1,3\text{-DNB}}$ / ppm | $C_{DNP}$ / ppm | $C_{PS}$ / ppm |
|---|---|---|---|---|---|
| Betrieb bei Nennlast (A) | - | - | 250 | 1 819 | 137 |
| Bsp. 1 (Vergleich) | *im Wes. gleich* | *im Wes. gleich* | 316 | 2388 | 215 |

(fortgesetzt)

| Nitrobenzol aus | $\dot{m}_1 / \dot{m}_2$ *im Vergleich zu A* | $\dot{m}_2 / \dot{m}_3$ *im Vergleich Zu A* | $C_{1,3\text{-DNB}} /$ ppm | $C_{DNP} /$ ppm | $C_{PS} /$ ppm |
|---|---|---|---|---|---|
| Bsp. 2 (erfindungsgemäß) | *im Wes. gleich* | *erniedrigt* | 284 | 1841 | 148 |
| Bsp. 3 (Vergleich) | *im Wes. gleich* | *im Wes. gleich* | 522 | 2 479 | 321 |
| Bsp. 4 (erfindungsgemäß) | *im Wes. gleich* | *erniedrigt* | 301 | 1945 | 162 |

**Anmerkungen:**

*im Wes.* = im Wesentlichen; c = Konzentration; DNB = Dinitrobenzol; DNP = Dinitrophenol; PS = Pikrinsäure.
Es ist unmittelbar ersichtlich, dass die Lastreduzierung in allen Fällen zu vermehrter Nebenproduktbildung führt. Jedoch ist der Anstieg des Nebenproduktgehalts bei erfindungsgemäßer Vorgehensweise erheblich geringer als bei den Vergleichsversuchen.

**Patentansprüche**

1. Kontinuierlich betriebenes Verfahren zur Herstellung von Nitrobenzol, umfassend die Nitrierung von Benzol (1) mit Salpetersäure (2) und Schwefelsäure (3), bei dem

   (i) der Nitrierung

   • ein Benzol (1) enthaltender Strom 10 eines Massenanteils an Benzol (1) $w_1$ mit einem Massenstrom $\dot{m}_{10}$,
   • ein Salpetersäure (2) enthaltender Strom 20 eines Massenanteils an Salpetersäure (2) $w_2$ mit einem Massenstrom $\dot{m}_{20}$ und
   • ein Schwefelsäure (3) enthaltender Strom 30 eines Massenanteils an Schwefelsäure $w_3$ mit einem Massenstrom $\dot{m}_{30}$

   zugeführt werden;
   (ii) $\dot{m}_{10}$ und $\dot{m}_{20}$ bei gegebenen Werten für $w_1$ und $w_2$ stets so gewählt werden, dass Benzol (1) im stöchiometrischen Überschuss zu Salpetersäure (2) vorliegt, wobei
   (iii) bei einer angestrebten Änderung der der Nitrierung über den Massenstrom $\dot{m}_{20}$ zugeführten Menge an Salpetersäure (2)

   • von einem Ausgangszustand A definiert durch einen Massenstrom an Salpetersäure (2) $\dot{m}_2 (A) = \dot{m}_{20} (A) \cdot w_2 (A)$, einem unter Beachtung von (ii) ausgewählten Massenstrom an Benzol (1) $\dot{m}_1 (A) = \dot{m}_{10} (A) \cdot w_1 (A)$ und einem Massenstrom an Schwefelsäure (3) $\dot{m}_3 (A) = \dot{m}_3 (A) \cdot w_3 (A)$,
   • in einen Endzustand E definiert durch einen Massenstrom an Salpetersäure (2) $\dot{m}_2 (E) = \dot{m}_{20} (E) \cdot w_2 (E)$, einem unter Beachtung von (ii) ausgewählten Massenstrom an Benzol (1) $\dot{m}_1 (E) = \dot{m}_{10} (E) \cdot w_1 (E)$ und einem Massenstrom an Schwefelsäure (3) $\dot{m}_3 (E) = \dot{m}_{30} (E) \cdot w_3 (E)$,

   der Massenstrom $\dot{m}_{20}$ und der Massenanteil $w_2$ so gewählt werden, dass sich der angestrebte Wert $\dot{m}_2 (E)$ einstellt, wobei mindestens eine solche Änderung der der Nitrierung über den Massenstrom $\dot{m}_{20}$ zugeführten Menge an Salpetersäure (2) durchgeführt wird, und wobei diese mindestens eine Änderung der der Nitrierung über den Massenstrom $\dot{m}_{20}$ zugeführten Menge an Salpetersäure (2)
   eine Erniedrigung auf einen Wert $\dot{m}_2 (E) < 0{,}95 \cdot \dot{m}_2 (A)$ für mehr als 0,50 Stunden ist,
   **dadurch gekennzeichnet, dass**

   das Verhältnis $\dot{m}_2 (E) / \dot{m}_3 (E)$ im Vergleich zum Verhältnis $\dot{m}_2 (A) / \dot{m}_3 (A)$ so erniedrigt wird, dass gilt:

   $$0{,}45 \cdot \dot{m}_2 (A) / \dot{m}_3 (A) \leq \dot{m}_2 (E) / \dot{m}_3 (E) \leq 0{,}97 \cdot \dot{m}_2 (A) / \dot{m}_3 (A)$$

und das Verhältnis $\dot{m}_1$ (E) / $\dot{m}_2$ (E) im Vergleich um Verhältnis $\dot{m}_1$ (A) / $\dot{m}_2$ (A) maximal so geändert wird, dass gilt:

$$0,98 \cdot \dot{m}_1\,(A)\,/\,\dot{m}_2\,(A) \leq \dot{m}_1\,(E)\,/\,\dot{m}_2\,(E) \leq 1,02 \cdot \dot{m}_1\,(A)\,/\,\dot{m}_2\,(A).$$

2. Verfahren gemäß Anspruch 1, bei welchem die Nitrierung von Benzol zu Nitrobenzol adiabatisch betrieben wird.

3. Verfahren gemäß Anspruch 2, bei welchem die Nitrierung von Benzol zu Nitrobenzol die folgenden Schritte umfasst:

(I) Nitrierung von Benzol mit Salpetersäure und Schwefelsäure unter Bildung von Nitrobenzol in einem Reaktor, wobei ein Benzol-haltiger Strom 10 mit einem Massenstrom $\dot{m}_{10}$, ein Salpetersäure-haltiger Strom 20 mit einem Massenstrom $\dot{m}_{20}$ und ein Schwefelsäure-haltiger Strom 30 mit einem Massenstrom $\dot{m}_{30}$ in den Reaktor eingetragen werden;
(II) Phasentrennung des Reaktionsgemisches aus Schritt (1) in einem Phasentrennapparat in eine wässrige, Schwefelsäure enthaltende Phase und eine organische, Nitrobenzol enthaltende Phase;
(III) Aufkonzentrierung der in Schritt (II) erhaltenen wässrigen Phase durch Verdampfung von Wasser in einem Verdampfungsapparat zu einer wässrigen Schwefelsäure-haltigen Phase mit erhöhter Schwefelsäurekonzentration, und wobei die aufkonzentrierte, Schwefelsäure-haltige, wässrige Phase in Schritt (I) zurückgeführt und als Bestandteil des Schwefelsäure-haltigen Stroms 30 eingesetzt wird;
(IV) mindestens zweistufiges Waschen der in Schritt (II) erhaltenen organischen, Nitrobenzol enthaltenden Phase und Abtrennung der wässrigen Phase nach jeder Stufe;
(V) Destillation der in der letzten Stufe von Schritt (IV) erhaltenen organischen, Nitrobenzol enthaltenden Phase unter Abtrennung von nicht umgesetztem Benzol, welches in Schritt (I) zurückgeführt und als Bestandteil des Benzol-haltigen Stroms 10 eingesetzt wird.

4. Verfahren gemäß Anspruch 3, bei welchem sich an Schritt (V) anschließt:

(VI) Aufarbeitung des Abwassers der ersten Waschstufe von Schritt (IV) umfassend Reinigung dieses Abwassers in einer Vorrichtung zur Destillation oder Strippung,
(VII) Aufarbeitung des Abwassers der zweiten Waschstufe von Schritt (IV) umfassend Reinigung dieses Abwassers in einer Vorrichtung zur Destillation oder Strippung, wobei der Vorrichtung zur Destillation oder Strippung eine Vorrichtung zur thermischen Druckzersetzung vor- und/oder nachgeschaltet sein kann.

5. Verfahren gemäß einem der Ansprüche 3 oder 4, bei welchem Schritt (IV) umfasst:

(IVa) Waschen der in Schritt (II) erhaltenen organischen, Nitrobenzol enthaltenen Phase in mindestens einer Wäsche und anschließende Phasentrennung in eine wässrige und eine organische, Nitrobenzol enthaltende Phase (erste Waschstufe);
(IVb) Waschen der in Schritt (IVa) erhaltenen organischen Phase in mindestens einer alkalischen Wäsche mit einer wässrigen Lösung einer Base und anschließende Phasentrennung in eine wässrige und eine organische, Nitrobenzol enthaltende Phase (zweite Waschstufe);
(IVc) Waschen der in Schritt (IVb) erhaltenen organischen Phase in mindestens einer neutralen Wäsche mit Wasser und anschließender Phasentrennung in eine wässrige und eine organische, Nitrobenzol enthaltende organische Phase (dritte Waschstufe).

6. Verfahren gemäß einem der vorstehenden Ansprüche, bei welchem das Verhältnis $\dot{m}_1$ (A) / $\dot{m}_2$ (A) im Bereich von 1,26 bis 1,74 liegt.

7. Verfahren gemäß einem der vorstehenden Ansprüche, bei welchem gilt:

$$0,40 \cdot \dot{m}_2(A) \leq \dot{m}_2\,(E) < 0,95 \cdot \dot{m}_2(A).$$

8. Verfahren gemäß Anspruch 7, bei welchem die folgenden Beziehungen gelten:

wenn $\dot{m}_2$ (E) im Bereich von $0,80 \cdot \dot{m}_2$ (A) bis $< 0,95 \cdot \dot{m}_2$ (A) liegt, wird $\dot{m}_2$ (E) / $\dot{m}_3$ (E) auf einen Wert im Bereich von $0,80 \cdot \dot{m}_2$ (A) / $\dot{m}_3$ (A) bis $0,97 \cdot \dot{m}_2$ (A) / $\dot{m}_3$ (A) eingestellt;

wenn $\dot{m}_2$ (E) im Bereich von 0,65 $\cdot$ $\dot{m}_2$ (A) bis < 0,80 $\cdot$ $\dot{m}_2$ (A) liegt, wird $\dot{m}_2$ (E) / $\dot{m}_3$ (E) auf einen Wert im Bereich von 0,65 $\cdot$ $\dot{m}_2$ (A) / $\dot{m}_3$ (A) bis < 0,80 $\cdot$ $\dot{m}_2$ (A) / $\dot{m}_3$ (A) eingestellt; und

wenn $\dot{m}_2$ (E) im Bereich von 0,40 $\cdot$ $\dot{m}_2$ (A) bis < 0,65 $\cdot$ $\dot{m}_2$ (A) liegt, wird $\dot{m}_2$ (E) / $\dot{m}_3$ (E) auf einen Wert im Bereich von 0,40 $\cdot$ $\dot{m}_2$ (A) / $\dot{m}_3$ (A) bis < 0,65 $\cdot$ $\dot{m}_2$ (A) / $\dot{m}_3$ (A) eingestellt.

9. Verfahren gemäß Anspruch 8, bei welchem gilt 0,98 $\cdot$ $\dot{m}_3$ (A) $\leq$ $\dot{m}_3$(E) $\leq$ 1,02 $\cdot$ $\dot{m}_3$ (A).

10. Verfahren gemäß einem der vorstehenden Ansprüche, bei welchem gilt $w_1$ (A) = $w_1$ (E) und/oder $w_2$ (A) = $w_2$ (E) und/oder $w_3$ (A) = $w_3$ (E).

11. Verfahren gemäß Anspruch 10, bei welchem gilt $w_1$ (A) = $w_1$ (E), $w_2$ (A) = $w_2$ (E) und $w_3$ (A) = $w_3$ (E).

**Claims**

1. Continuously operated process for preparing nitrobenzene, comprising the nitration of benzene (1) with nitric acid (2) and sulfuric acid (3), in which

   (i) the nitration is supplied with

   - a stream 10 containing benzene (1) and having a proportion by mass of benzene (1) $w_1$ with a mass flow rate of $\dot{m}_{10}$,
   - a stream 20 containing nitric acid (2) and having a proportion by mass of nitric acid (2) $w_2$ with a mass flow rate of $\dot{m}_{20}$ and
   - a stream 30 containing sulfuric acid (3) and having a proportion by mass of sulfuric acid $w_3$ with a mass flow rate of $\dot{m}_{30}$;

   (ii) $\dot{m}_{10}$ and $\dot{m}_{20}$, for given values of $w_1$ and $w_2$, are always chosen such that benzene (1) is in a stoichiometric excess relative to nitric acid (2), where
   (iii) in the event of a desired change in the amount of nitric acid (2) supplied to the nitration via the mass flow $\dot{m}_{20}$

   - from a starting state A defined by a mass flow rate of nitric acid (2) $\dot{m}_2$ (A) = $\dot{m}_{20}$ (A) $\bullet$ $w_2$(A), a mass flow rate of benzene (1) $\dot{m}_1$ (A) = $\dot{m}_{10}$ (A) $\bullet$ $w_1$ (A) selected with regard to (ii) and a mass flow rate of sulfuric acid (3) $\dot{m}_3$ (A) = $\dot{m}_{30}$ (A) $\bullet$ $w_3$(A),
   - to a final state E defined by a mass flow rate of nitric acid (2) $\dot{m}_2$ (E) = $\dot{m}_{20}$ (E) $\bullet$ $w_2$(E), a mass flow rate of benzene (1) $\dot{m}_1$(E) = $\dot{m}_{10}$(E) $\bullet$ $w_1$ (E) selected with regard to (ii) and a mass flow rate of sulfuric acid (3) $\dot{m}_3$(E) = $\dot{m}_{30}$ (E) $\bullet$ $w_3$(E),

     the mass flow rate $\dot{m}_{20}$ and the proportion by mass $w_2$ are chosen such that the desired value $\dot{m}_2$ (E) is established, where at least one such change in the amount of nitric acid (2) supplied to the nitration via the mass flow $\dot{m}_{20}$ is conducted, and where this at least one change in the amount of nitric acid (2) supplied to the nitration via the mass flow $\dot{m}_{20}$
     is a <u>decrease</u> to a value $\dot{m}_2$ (E) < 0.95 $\bullet$ $\dot{m}_2$ (A) for more than 0.50 hour,
     **characterized in that**
     the ratio $\dot{m}_2$ (E) / $\dot{m}_3$ (E) is decreased compared to the ratio $\dot{m}_2$ (A) / $\dot{m}_3$ (A) such that:

$$0.45 \bullet \dot{m}_2(A) \ / \ \dot{m}_3(A) \ \leq \ \dot{m}_2(E) \ / \ \dot{m}_3(E) \ \leq \ 0.97 \bullet \dot{m}_2(A) \ / \ \dot{m}_3(A)$$

   and the ratio $\dot{m}_1$ (E) / $\dot{m}_2$ (E) is altered to a maximum degree compared to the ratio $\dot{m}_1$ (A) / $\dot{m}_2$ (A) such that:

$$0.98 \bullet \dot{m}_1(A) \ / \ \dot{m}_2(A) \ \leq \ \dot{m}_1(E) \ / \ \dot{m}_2(E) \ \leq \ 1.02 \bullet \dot{m}_1(A) \ / \ \dot{m}_2(A).$$

2. Process according to Claim 1, in which the nitration of benzene to nitrobenzene is operated adiabatically.

**3.** Process according to Claim 2, in which the nitration of benzene to nitrobenzene comprises the following steps:

(I) nitrating benzene with nitric acid and sulfuric acid to form nitrobenzene in a reactor, with introduction of a benzene-containing stream 10 with a mass flow rate of $\dot{m}_{10}$, a nitric acid-containing stream 20 with a mass flow rate of $\dot{m}_{20}$ and a sulfuric acid-containing stream 30 with a mass flow rate of $\dot{m}_{30}$ into the reactor;
(II) separating the phases of the reaction mixture from step (I) in a phase separation apparatus into an aqueous, sulfuric acid-containing phase and an organic, nitrobenzene-containing phase;
(III) concentrating the aqueous phase obtained in step (II) by evaporating water in an evaporation apparatus to give an aqueous sulfuric acid-containing phase having elevated sulfuric acid concentration, with recycling of the concentrated sulfuric acid-containing aqueous phase into step (I) and use thereof as a constituent of the sulfuric acid-containing stream 30;
(IV) washing, in at least two stages, the organic, nitrobenzene-containing phase obtained in step (II) and separating the aqueous phase off after each stage;
(V) distilling the organic, nitrobenzene-containing phase obtained in the last stage of step (IV) with removal of unconverted benzene which is recycled into step (I) and used as a constituent of the benzene-containing stream 10.

**4.** Process according to Claim 3, in which step (V) is followed by:

(VI) working up the wastewater from the first wash stage of step (IV), comprising cleaning this wastewater in an apparatus for distillation or stripping,
(VII) working up the wastewater from the second wash stage of step (IV), comprising cleaning this wastewater in an apparatus for distillation or stripping, where an apparatus for thermal pressure decomposition may be connected up- and/or downstream of the apparatus for distillation or stripping.

**5.** Process according to either of Claims 3 and 4, in which step (IV) comprises:

(IVa) washing the organic, nitrobenzene-containing phase obtained in step (II) in at least one wash, then separating the phases into an aqueous phase and an organic, nitrobenzene-containing phase (first wash stage);
(IVb) washing the organic phase obtained in step (IVa) in at least one alkaline wash with an aqueous solution of a base, then separating the phases into an aqueous phase and an organic, nitrobenzene-containing phase (second wash stage);
(IVc) washing the organic phase obtained in step (IVb) in at least one neutral wash with water, then separating the phases into an aqueous phase and an organic, nitrobenzene-containing organic phase (third wash stage).

**6.** Process according to any of the preceding claims, in which the ratio $\dot{m}_1$ (A) / $\dot{m}_2$ (A) is in the range from 1.26 to 1.74.

**7.** Process according to any of the preceding claims, in which:

$$0.40 \cdot \dot{m}_2(A) \leq \dot{m}_2(E) < 0.95 \cdot \dot{m}_2(A).$$

**8.** Process according to Claim 7, in which the following relationships are applicable:

when $\dot{m}_2$ (E) is in the range from 0.80 $\cdot$ $\dot{m}_2$ (A) to < 0.95 $\cdot$ $\dot{m}_2$ (A), $\dot{m}_2$ (E) / $\dot{m}_3$ (E) is adjusted to a value in the range from 0.80 $\cdot$ $\dot{m}_2$ (A) / $\dot{m}_3$ (A) to 0.97 $\cdot$ $\dot{m}_2$ (A) / $\dot{m}_3$ (A) ;
when $\dot{m}_2$ (E) is in the range from 0.65 $\cdot$ $\dot{m}_2$ (A) to < 0.80 $\cdot$ $\dot{m}_2$ (A), $\dot{m}_2$ (E) / $\dot{m}_3$ (E) is adjusted to a value in the range from 0.65 $\cdot$ $\dot{m}_2$ (A) / $\dot{m}_3$ (A) to < 0.80 $\cdot$ $\dot{m}_2$ (A) / $\dot{m}_3$ (A) ;
and
when $\dot{m}_2$ (E) is in the range from 0.40 $\cdot$ $\dot{m}_2$ (A) to < 0.65 $\cdot$ $\dot{m}_2$ (A), $\dot{m}_2$ (E) / $\dot{m}_3$ (E) is adjusted to a value in the range from 0.40 $\cdot$ $\dot{m}_2$ (A) / $\dot{m}_3$ (A) to < 0.65 $\cdot$ $\dot{m}_2$ (A) / $\dot{m}_3$ (A) .

**9.** Process according to Claim 8, in which 0.98 $\cdot$ $\dot{m}_3$ (A) $\leq$ $\dot{m}_3$ (E) $\leq$ 1.02 $\cdot$ $\dot{m}_3$ (A) .

**10.** Process according to any of the preceding claims, in which $w_1$ (A) = $w_1$ (E) and/or $w_2$ (A) = $w_2$ (E) and/or $w_3$ (A) = $w_3$ (E).

**11.** Process according to Claim 10, in which $w_1$ (A) = $w_1$ (E), $w_2$ (A) = $w_2$ (E) and $w_3$ (A) = $w_3$ (E).

**Revendications**

1. Procédé fonctionnant en continu pour la production de nitrobenzène, comprenant la nitration de benzène (1) avec de l'acide nitrique (2) et de l'acide sulfurique (3), selon lequel

   (i) on amène à la nitration

      • un courant 10 contenant du benzène (1) d'une fraction massique de benzène (1) $w_1$ ayant un débit massique $\dot{m}_{10}$,
      • un courant 20 contenant de l'acide nitrique (2) d'une fraction massique d'acide nitrique (2) $w_2$ ayant un débit massique $\dot{m}_{20}$ et
      • un courant 30 contenant de l'acide sulfurique (3) d'une fraction massique d'acide sulfurique $w_3$ ayant un débit massique $\dot{m}_{30}$ ;

   (ii) $\dot{m}_{10}$ et $\dot{m}_{20}$ sont toujours choisis, pour des valeurs données de $w_1$ et $w_2$, de telle sorte que le benzène (1) soit présent en excès stoechiométrique par rapport à l'acide nitrique (2),
   (iii) si l'on souhaite modifier la quantité d'acide nitrique (2) amenée à la nitration par le biais du débit massique $\dot{m}_{20}$

      - à partir d'un état initial A défini par un débit massique d'acide nitrique (2) $\dot{m}_2 (A) = \dot{m}_{20} (A) \cdot W_2 (A)$, un débit massique de benzène (1) $\dot{m}_1 (A) = \dot{m}_{10} (A) \cdot w_1 (A)$ choisi en tenant compte de (ii) et un débit massique d'acide sulfurique (3) $\dot{m}_3 (A) = \dot{m}_{30} (A) \cdot w_3 (A)$,
      - dans un état final E défini par un débit massique d'acide nitrique (2) $\dot{m}_2 (E) = \dot{m}_{20} (E) \cdot w_2 (E)$, un débit massique de benzène (1) $\dot{m}_1 (E) = \dot{m}_{10} (E) \cdot w_1 (E)$ choisi en tenant compte de (ii) et un débit massique d'acide sulfurique (3) $\dot{m}_3 (E) = \dot{m}_{30} (E) \cdot w_3 (E)$,

         le débit massique $\dot{m}_{20}$ et la fraction massique $w_2$ sont choisis de telle sorte que la valeur souhaitée $\dot{m}_2(E)$ s'ajuste, au moins une telle modification de la quantité d'acide nitrique (2) amenée à la nitration par le biais du débit massique $\dot{m}_{20}$ étant effectuée, et cette au moins une modification de la quantité d'acide nitrique (2) amenée à la nitration par le biais du débit massique $\dot{m}_{20}$ étant un abaissement à une valeur $\dot{m}_2 (E) < 0,95 \cdot \dot{m}_2 (A)$ pendant plus de 0,50 heure,
         **caractérisé en ce que**
         le rapport $\dot{m}_2 (E) / \dot{m}_3 (E)$ est abaissé en comparaison du rapport $\dot{m}_2(A) / \dot{m}_3(A)$ de telle sorte que :

$$0,45 \cdot \dot{m}_2 (A) \ / \ \dot{m}_3 (A) \ \leq \ \dot{m}_2 (E) \ / \ \dot{m}_3 (E) \ \leq \ 0,97 \cdot \dot{m}_2 (A) \ / \ \dot{m}_3 (A)$$

         et le rapport $\dot{m}_1(E)/ \dot{m}_2(E)$ est modifié au maximum en comparaison du rapport $\dot{m}_1(A) / \dot{m}_2(A)$ de telle sorte que :

$$0,98 \cdot \dot{m}_1 (A) \ / \ \dot{m}_2 (A) \ \leq \ \dot{m}_1 (E) \ / \ \dot{m}_2 (E) \ \leq \ 1,02 \cdot \dot{m}_1 (A) \ / \ \dot{m}_2 (A).$$

2. Procédé selon la revendication 1, selon lequel la nitration de benzène en nitrobenzène fonctionne de manière adiabatique.

3. Procédé selon la revendication 2, selon lequel la nitration de benzène en nitrobenzène comprend les étapes suivantes :

   (I) la nitration de benzène avec de l'acide nitrique et de l'acide sulfurique pour former du nitrobenzène dans un réacteur, un courant 10 contenant du benzène ayant un débit massique $\dot{m}_{10}$, un courant 20 contenant de l'acide nitrique ayant un débit massique $\dot{m}_{20}$ et un courant 30 contenant de l'acide sulfurique ayant un débit massique $\dot{m}_{30}$ étant introduits dans le réacteur ;
   (II) la séparation de phases du mélange réactionnel de l'étape (I) dans un appareil de séparation de phases en une phase aqueuse contenant de l'acide sulfurique et une phase organique contenant du nitrobenzène ;
   (III) la concentration de la phase aqueuse obtenue dans l'étape (II) par évaporation d'eau dans un appareil d'évaporation en une phase aqueuse contenant de l'acide sulfurique ayant une concentration accrue d'acide sulfurique, et la phase aqueuse concentrée contenant de l'acide sulfurique étant recyclée dans l'étape (I) et utilisée en tant que constituant du courant 30 contenant de l'acide sulfurique ;

(IV) le lavage en au moins deux étapes de la phase organique contenant du nitrobenzène obtenue dans l'étape (II) et la séparation de la phase aqueuse après chaque étape ;

(V) la distillation de la phase organique contenant du nitrobenzène obtenue dans la dernière étape de l'étape (IV) avec séparation de benzène n'ayant pas réagi, qui est recyclé dans l'étape (I) et utilisé en tant que constituant du courant 10 contenant du benzène.

4. Procédé selon la revendication 3, selon lequel l'étape (V) est suivie par :

(VI) le traitement de l'eau résiduaire de la première étape de lavage de l'étape (IV) comprenant la purification de cette eau résiduaire dans un dispositif de distillation ou d'extraction,

(VII) le traitement de l'eau résiduaire de la deuxième étape de lavage de l'étape (IV) comprenant la purification de cette eau résiduaire dans un dispositif de distillation ou d'extraction, le dispositif de distillation ou d'extraction pouvant être précédé et/ou suivi d'un dispositif de décomposition thermique sous pression.

5. Procédé selon l'une quelconque des revendications 3 ou 4, selon lequel l'étape (IV) comprend :

(IVa) le lavage de la phase organique contenant du nitrobenzène obtenue dans l'étape (II) dans au moins un lavage, puis la séparation de phases en une phase aqueuse et une phase organique contenant du nitrobenzène (première étape de lavage) ;

(IVb) le lavage de la phase organique obtenue dans l'étape (IVa) dans au moins un lavage alcalin avec une solution aqueuse d'une base, puis la séparation de phases en une phase aqueuse et une phase organique contenant du nitrobenzène (deuxième étape de lavage) ;

(IVc) le lavage de la phase organique obtenue dans l'étape (IVb) dans au moins un lavage neutre avec de l'eau, puis la séparation de phases en une phase aqueuse et une phase organique contenant du nitrobenzène (troisième étape de lavage).

6. Procédé selon l'une quelconque des revendications précédentes, dans lequel le rapport $\dot{m}_1$ (A) / $\dot{m}_2$ (A) se situe dans la plage allant de 1,26 à 1,74.

7. Procédé selon l'une quelconque des revendications précédentes, dans lequel :

$$0,40 \cdot \dot{m}_2(A) \leq \dot{m}_2(E) < 0,95 \cdot \dot{m}_2(A).$$

8. Procédé selon la revendication 7, dans lequel les relations suivantes s'appliquent :

lorsque $\dot{m}_2$ (E) se situe dans la plage allant de 0,80 • $\dot{m}_2$ (A) à < 0,95 • $\dot{m}_2$ (A), $\dot{m}_2$ (E) / $\dot{m}_3$ (E) est ajusté à une valeur dans la plage allant de 0,80 • $\dot{m}_2$ (A) / $\dot{m}_3$ (A) à 0,97 • $\dot{m}_2$ (A) / $\dot{m}_3$ (A) ;

lorsque $\dot{m}_2$ (E) se situe dans la plage allant de 0,65 • $\dot{m}_2$ (A) à < 0,80 • $\dot{m}_2$ (A), $\dot{m}_2$ (E) / $\dot{m}_3$ (E) est ajusté à une valeur dans la plage allant de 0, 65 • $\dot{m}_2$ (A) / $\dot{m}_3$ (A) à < 0,80 • $\dot{m}_2$ (A) / $\dot{m}_3$ (A) ;

et

lorsque $\dot{m}_2$ (E) se situe dans la plage allant de 0,40 • $\dot{m}_2$ (A) à < 0, 65 • $\dot{m}_2$ (A), $\dot{m}_2$ (E) / $\dot{m}_3$ (E) est ajusté à une valeur dans la plage allant de 0,40 • $\dot{m}_2$ (A) / $\dot{m}_3$ (A) à < 0, 65 • $\dot{m}_2$ (A) / $\dot{m}_3$ (A).

9. Procédé selon la revendication 8, dans lequel 0,98

• $\dot{m}_3$ (A) ≤ $\dot{m}_3$ (E) ≤ 1,02 • $\dot{m}_3$ (A).

10. Procédé selon l'une quelconque des revendications précédentes, dans lequel $w_1$ (A) = $w_1$ (E) et/ou $w_2$ (A) = $w_2$ (E) et/ou $w_3$ (A) = $w_3$ (E).

11. Procédé selon la revendication 10, dans lequel $w_1$ (A) - $w_1$ (E), $w_2$ (A) = $w_2$ (E) et $w_3$ (A) = $w_3$ (E).

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0436443 B1
- EP 0771783 B1
- US 6562247 B2
- EP 1816117 A1
- US 4091042 A
- US 5763697 A
- DE 2821571 A1
- WO 2015197521 A1
- WO 2014177450 A1
- WO 2014016292 A1

- WO 2014016290 A1
- EP 0156199 B1
- EP 0708076 B1
- EP 1291078 A2
- DE 102008048713 A1
- EP 2354117 A1
- EP 1593654 A1
- EP 1132347 A2
- WO 2012013678 A2
- EP 1593654 B1